# EUROPEAN PATENT APPLICATION

(11) **EP 2 189 451 A1**
(43) Date of publication of application: **26.05.2010**
(21) Application number: 08800554.1
(22) Date of filing: 01.09.2008
(51) Int. Cl.: C07D 265/30, C07D 413/12, C07D 471/08, A61K 31/46, A61K 31/5377, A61P 1/00

(54) **BENZAMIDE DERIVATIVES, THEIR PREPARATION AND USES IN MEDICINE THEREOF**

(30) Priority: 11.09.2007 CN 200710154407; 30.07.2008 CN 200810129978
(71) Applicant: Jiangsu Hengrui Medicine Co., Ltd., Lianyungang, Jiangsu 222002 (CN); Shanghai Hengrui Pharmaceutical Co. Ltd., Minhang District Shanghai 200245 (CN)
(72) Inventor: LEI, Xinsheng, Shanghai 200245 (CN); TANG, Peng Cho, Shanghai 200245 (CN); SUN, Piaoyang, Shanghai 200245 (CN); DONG, Zheng, Shanghai 200245 (CN)
(74) Representative: Ahner, Francis
(86) International application number: PCT/CN2008/001557
(87) International publication number: WO 2009/033360

(57) **Abstract**

The present invention discloses novel benzamide derivatives represented by general formula (I), their preparation, pharmaceutical compositions containing the derivatives and their use as medicament, especially as a 5-HT₄ stimulator, wherein the definition of each substituent of the general formula (I) is the same as defined in the description.

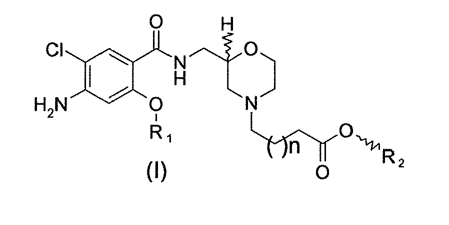

## Description

### FIELD OF THE INVENTION

The present invention relates to novel benzamide derivatives, their preparation and pharmaceutical compositions containing them and the use of such derivatives as medicament, especially as a 5-HT₄ receptor active agonist.

### BACKGROUND OF THE INVENTION

5-HT is a neurotransmitter that is widely distributed throughout body, both in central nervous system and in peripheral systems. At least seven subtypes of 5-HT receptors have been identified and interactions of 5-HT with these different receptors are associated with a variety of physiological functions. There has been, therefore, substantial interest in investigating 5-HT receptor subtypes.

It has been reported that 5-HT₄ receptor agonists are useful for treatment of a variety of diseases such as gastroesophageal reflux diseases, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

5-HT₄ receptor agonists comprise three types including: benzamides , benzimidazoles and indole alkyl amine derivatives. As one class, the benzamide derivatives have several prominent pharmacological actions, due to their effects on the neuronal systems which are modulated by the neurotransmitter serotonin. The role of serotonin, and thus the pharmacology of the benzamide derivatives, is broadly implicated in a variety of disease conditions. Therefore, research has been focused on the production and storage sites of serotonin as well as the location of serotonin receptors in the human body in order to determine the relationship between these sites and various disease states or conditions.

Cisapride and Mosapride, which are commonly used in clinic, are novel benzamide drugs.

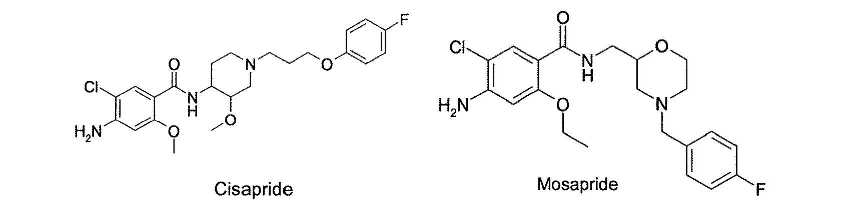

Benzamide drugs promote the release of acetylcholine, enhance the role of cholinergic and strengthen the gastrointestinal smooth muscle motility and contraction mainly by stimulating the pre-and post-synaptic neurons 5-HT₄ receptor on intestinal muscle. In large doses, these drugs can be effective central anti-emetic agents as 5-HT₃ receptor antagonist. In normal doses, these drugs control vomiting through the promotion of gastrointestinal motility. These drugs can improve the esophageal smooth muscle tension and promote esophageal peristalsis motility, improve the coordination of antrum and duodenal, promote gastric emptying, promote and enhance the function of small bowel movements, and increase the proximal colon emptying. They are considered as the entire gastrointestinal dynamic drugs promoting whole digestive tract smooth muscle motility, and are mainly used in patients suffering from reflux esophagitis, functional dyspepsia, gastroparesis, postoperative gastrointestinal paralysis, functional constipation and pseudo-obstruction. However, cisapride shows mildly antagonistic 5-HT₃ and anti-D2 receptor activity, it may cause cardiac adverse reactions and prolong Q-T interval. Patients with serious heart attack, prolonging Q-T interval shown by electrocardiogram, low blood potassium and low magnesium are prohibited from this drug. People with liver and kidney damage, lactating women and children are not recommended to use this drug. Due to the ability of increasing the absorption of diazepam, alcohol, acenocoumarol, cimetidine, ranitidine, anticholinergic drugs may antagonize the effect of cisapride to promote gastrointestinal peristalsis. The interactions among these drugs should be noted. For example, dosage should be reduced when diarrhoea occurs; attention should be paid to monitor clotting time in anticoagulant therapy. Mosapride is a selective gastrointestinal drug of 5-HT₄ receptor agonist, not the D2 receptor antagonis. It does not cause Q-T interval prolongation, and could reduce adverse reactions including mainly malaise, dizziness, water stool, mild abdominal pain etc. Compared with cisapride, it is commonly used and shows equivalent efficacy. Prucalopride, as a benzimidazole drug, has a high selectivity and specificity for 5-HT₄ receptor. It increases the release of cholinergic neurotransmitter, stimulates peristalsis reflex, enhance colon contraction, accelerates gastric emptying, and promotes digestive tract motility It can effectively alleviate the symptoms of constipation patients, and is mainly used for the treatment of constipation, weak gastrointestinal peristalsis and intestinal pseudo-obstruction.

WO2005068461 discloses a series of benzamide derivatives as 5-HT₄ receptor agonists, especially the compound having the formula:

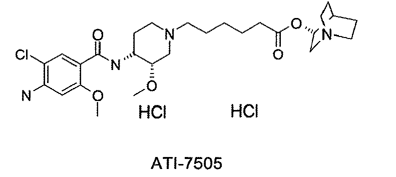

ATI-7505 is a stereoisomeric esterified cisapride analog, for safe and effective treatment of various gastrointestinal disorders including gastroparesis, gastroesophageal reflux and related disease conditions. ATI-7505 is useful for treatment or prevention of gastroesophageal reflux disease, and can also greatly reduce the side effects of Cisapride including diarrhea, abdominal cramps, blood pressure and heart rate increase, and so on.

In addition, the compounds and pharmaceutical compositions described in the patent can also be used for treatment of vomiting and other diseases such as dyspepsia, gastroesophageal reflux, constipation, postoperative intestinal infarction, and false intestinal obstruction. In the course of treatment, they may also reduce the side effects of cisapride.

As a ligand for the 5-HT₄ receptor, ATI-7505 can be used to treat disease conditions mediated by this receptor. The receptor is located in several areas of the central nervous system and the modulation of the receptor could achieve desired modulations of the CNS.

The ester section of ATI-7505 will not affect the treatment capacity of the compound, instead, it makes the compound easy for degradation by serum and/or Plasma EST. So it can evade the cytopigment P450 drug detoxification system which is related to the side effects caused by cisapride, and reduce the occurrence of side effects.

Excellent 5-HT₄ receptor agonists should be highly bound to 5-HT₄ receptor with almost no affinities to other receptors, and have great agonist activities. They should be well gastrointestinal absorbed, stably metabolised and have desirable pharmacokinetics properties. When targeting the receptor in the central nervous system, they should be capable of passing the blood-brain barrier. When selectively targeting peripheral nervous system receptors, they should be blocked by the blood-brain barrier. Meanwhile, they should be non-toxic and have very few side effects. In addition, the ideal candidates of the drug should exist in a stable, non-hygroscopic and easy-to-prepare physical form.

Based on the current 5-HT₄ receptor agonists, the present invention involves a series of benzamide derivatives which are more effective, safer and have fewer side effects.

### SUMMARY OF THE INVENTION

The present invention relates to compounds having the formula (I) or pharmaceutically acceptable salts thereof: 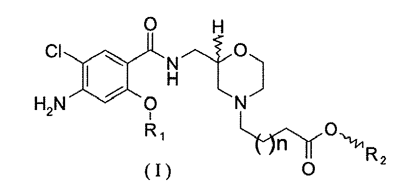 wherein:
R₁ is alkyl, preferably, methyl, ethyl or isopropyl;
R₂ is selected from the group consisting of hydrogen, alkyl, cycloalkyl and heterocyclo alkyl, preferably, hydrogen, alkyl or heterocyclo alkyl, and more preferably, 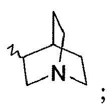 and
n is an integer from 1 to 5, preferably, 1 to 3.

Further, the present invention includes compounds having the formula (IA) or pharmaceutically acceptable salts thereof:

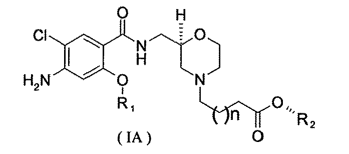

Further, the present invention includes compounds having the formula (IB) or pharmaceutically acceptable salts thereof:

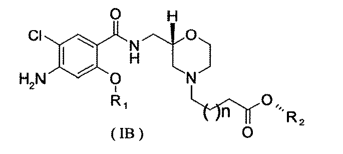

In another aspect, the present invention relates to compounds having the formula (I) or pharmaceutically acceptable salts thereof, wherein the compounds having the formula (I) are present in a pharmaceutically acceptable free-form or the forms of acid addition salts, wherein the said salts are those formed from the compounds according to the present invention with the acids selected from the group consisting of hydrochloric acid, methanesulfonic acid, sulfuric acid, tartaric acid, citric acid, acetic acid and trifluoroacetic acid.

Specifically, the compounds having the formula (I) according to the present invention include, but not limited to the following:

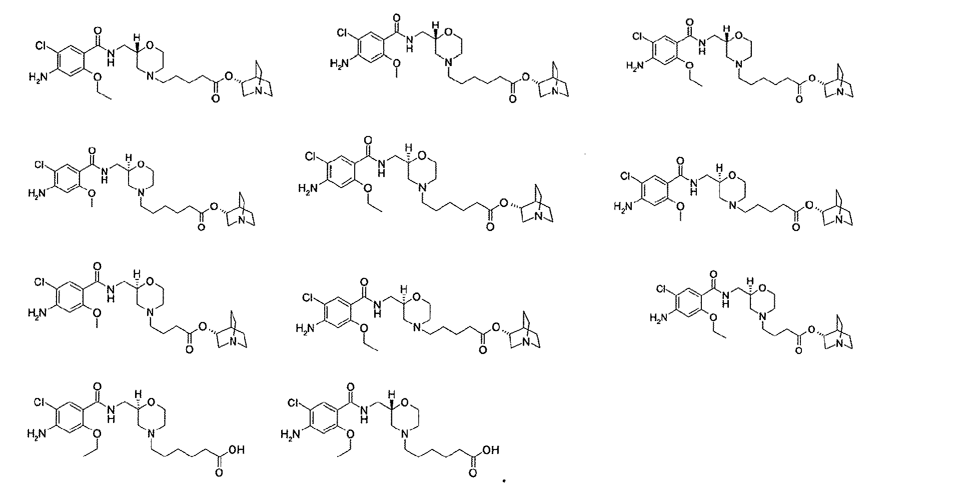

The present invention relates to the process for preparing compounds having the formula (I), wherein the process comprises the steps of: 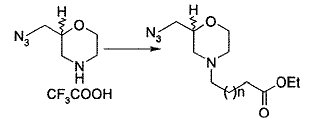 coupling azidomethyl-morpholine trifluoroacetate with Br-CH₂-(CH₂)n-CH₂-C(O)OEt under alkaline conditions to obtain N-substituted azidomethyl-morpholine; 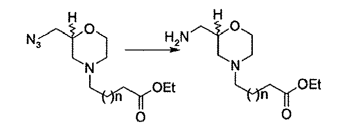 reducing N-substituted azidomethyl-morpholine by hydrogenation to obtain N-substituted aminomethyl-morpholine; 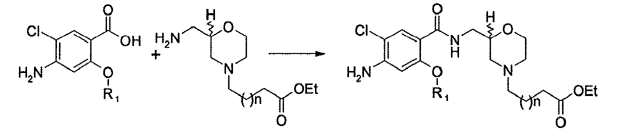 reacting N-substituted aminomethyl-morpholine with substituted benzoic acid in the presence of the condensation reagent 1-ethyl-3-(3-dimethyl-propyl) carbodiimide hydrochloride at room temperature to obtain benzamide derivatives; 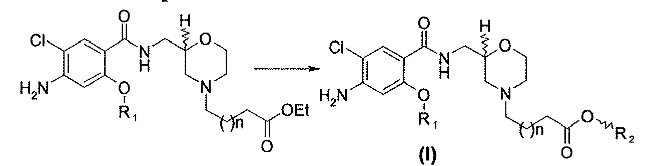 reacting benzamide derivatives in toluene with R₂-OH by transesterification, or hydrolyzing under alkaline conditions to obtain compounds having the formula (I).

Further, the present invention relates to the process for preparing compounds having the formula (I), wherein the process comprises the steps of: 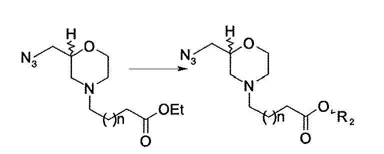 reacting N-substituted azidomethyl-morpholine in toluene with R₂-OH by transesterification to obtain the transesterification product; 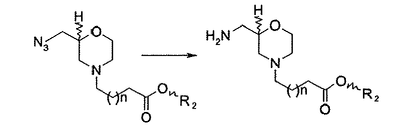 reducing the transesterification product by hydrogenation to obtain N-substituted aminomethyl-morpholine; 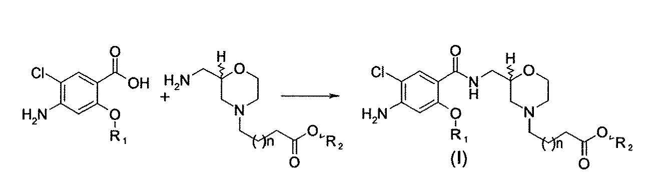 reacting N-substituted aminomethyl-morpholine with substituted benzoic acid in the presence of the condensation reagent 1-ethyl-3-(3-dimethyl-propyl)carbodiimide hydrochloride at room temperature, or further hydrolyzing under alkaline conditions to obtain compounds having the formula (I).

Further, the present invention relates to a pharmaceutical composition comprising compounds having the formula (I) or salts thereof in an effective therapeutic amount, as well as pharmaceutically acceptable carriers.

Furthermore, the present invention also relates to the use of the compounds having the formula (I) or pharmaceutically acceptable salts thereof in the preparation of a medicament for the treatment of the diseases selected from the group consisting of gastroesophageal reflux disease, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

Furthermore, the present invention relates to the use of the pharmaceutical composition according to the present invention in the preparation of a medicament for the treatment of the diseases selected from the group consisting of gastroesophageal reflux disease, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

Unless otherwise stated, the following terms used in the specification and claims have the meanings discussed below.

"Alkyl" refers to saturated aliphatic hydrocarbon radicals including straight chain or branched chain radical having 1 to 20 carbon atoms. The alkyl groups having 1 to 10 carbon atoms, such as methyl, ethyl, propyl, 2-propyl, n-butyl, iso-butyl, tert-butyl, pentyl, and the like, are preferable. The lower alkyl groups having 1 to 4 carbon atoms, such as methyl, ethyl, propyl, 2-propyl, n-butyl, iso-butyl, or tert-butyl, and the like, are more preferable. The alkyl group may be substituted or unsubstituted. When the alkyl group is substituted, the substituent is preferably one or more radicals independently selected from cycloalkyl or heterocyclo alkyl.

"Cycloalkyl" refers to an all-carbon ring radical having 3 to 8 membered monocyclic ring, 5-membered/6-membered or 6-membered/6-membered fused bicyclic ring or multicyclic fused ring (a "fused" ring system means that each ring in the system shares an adjacent pair of carbon atoms with other ring in the system) wherein one or more rings may contain one or more double bonds while none of the rings has a completely conjugated π-electron system. Examples of cycloalkyl groups are cyclopropyl, cyclobutyl, cyclopentyl, cyclopentenyl, cyclohexyl, cyclohexadienyl, adamantyl, cycloheptyl, cycloheptatrienyl, and the like.

"Heterocyclo alkyl" refers to a monocyclic or fused ring radical having 5 to 9 ring atoms, wherein one or two ring atoms are heteroatoms selected from N, O, or S(O)n (n is an integer from 0 to 2), the remaining ring atoms being C. The rings may also have one or more double bonds while have no completely conjugated π-electron system. The unsubstituted heterocyclo alkyl groups include but not limited to pyrrolidinyl, piperidyl, piperazyl, morpholinyl, thiomorpholinyl, homopiperazinyl and the like. In one embodiment, the heterocyclo alkyl group is substituted or unsubstituted. The alkynyl group may be substituted or unsubstituted. When the alkynyl group is substituted, the substituent is preferably one or more radicals independently selected from cycloalkyl or heterocyclo alkyl.

"Optional" or "optionally" means that the subsequently described event or circumstance may but may not necessarily occur, and the description includes situations where the event or circumstance may or may not occur. For example, "aryl group optionally substituted with an alkyl group" means that the alkyl group may but may not necessarily be present, and the description includes situations where the aryl group is substituted with an alkyl group and situations where the aryl group is not substituted with an alkyl group.

### DETAILED DESCRIPTION OF THE INVENTION

### SYNTHESIS METHOD OF THE INVENTIVE COMPOUNDS

In order to achieve the object of the present invention, the present invention applies the following technical solution:

A process for preparing compounds having the formula (I) or salts thereof, comprising the steps of: 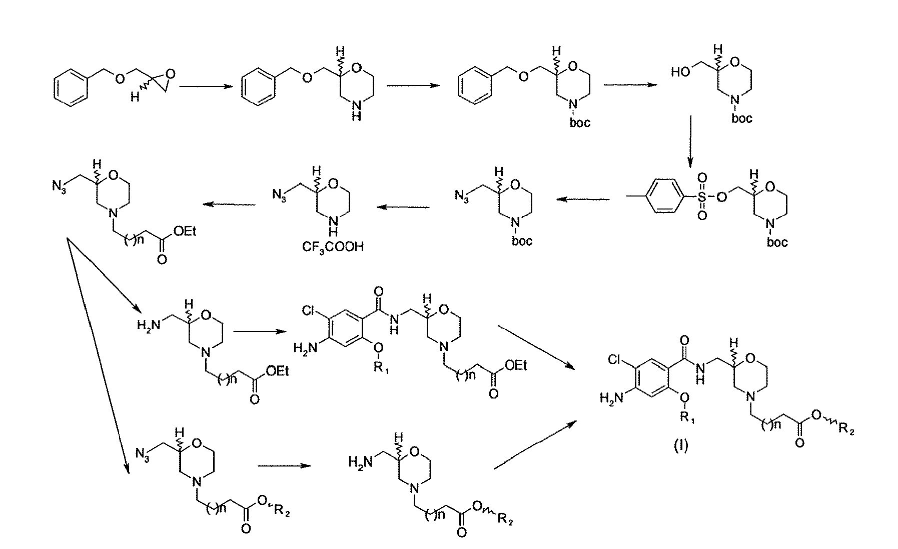 wherein, reacting the starting material benzyl glycidyl ether in methanol with mono-(2-aminoethyl) sulfate under alkaline conditions to obtain 2-(benzyloxymethyl) morpholine; reacting 2-(benzyloxymethyl)morpholine with di-tert-butyl dicarbonate under alkaline conditions at room temperature to obtain N-tert-butoxycarbonyl protected 2-(benzyloxymethyl) morpholine; reducing the protected 2-(benzyloxymethyl) morpholine in methanol by hydrogenation to obtain N-protected 2-(hydroxymethyl) morpholine; reacting the N-protected 2-(hydroxymethyl) morpholine with p-toluenesulfonyl chloride and triethylamine at room temperature to obtain N-protected 2-(p-toluenesulfonyloxymethyl) morpholine; reacting the N-protected 2-(p-toluenesulfonyloxymethyl) morpholine in N,N-dimethylformamide with sodium azide in an oil bath to obtain N-protected azidomethyl morpholine; reacting the N-protected azidomethyl-morpholine in dichloromethane with trifluoroacetic acid to remove the protecting group to obtain azidomethyl-morpholine trifluoroacetate; coupling the azidomethyl-morpholine trifluoroacetate with Br-CH₂-(CH₂)n-CH₂-C(O)OEt under alkaline conditions to obtain N-substituted azidomethyl-morpholine; reducing the N-substituted azidomethyl-morpholine by hydrogenation to obtain N-substituted aminomethyl-morpholine; reacting the N-substituted aminomethyl-morpholine with substituted benzoic acids in the presence of the condensation reagent 1-ethyl-3-(3-dimethyl-propyl)carbodiimide hydrochloride at room temperature to obtain benzamide derivatives; reacting the benzamide derivatives in toluene with R₂-OH by transesterification, or hydrolyzing under alkaline conditions to obtain compounds having the formula (I).

Alternatively, reacting the N-substituted azidomethyl-morpholine in toluene with R₂-OH by transesterification to obtain the transesterification products; reducing the transesterification product by hydrogenation to obtain N-substituted aminomethyl-morpholine; reacting the N-substituted aminomethyl-morpholine with substituted benzoic acid in the presence of the condensation reagent 1-ethyl-3-(3-dimethyl-propyl)carbodiimide hydrochloride at room temperature, or further hydrolyzing under alkaline conditions to obtain compounds having the formula (I), wherein:
R₁ is alkyl;
R₂ is selected from the group consisting of hydrogen, alkyl, cycloalkyl and heterocyclic alkyl ; and
n is an integer from 1 to 5.

The present invention relates to a pharmaceutical composition comprising a compound or salts thereof in an effective therapeutic amount, as well as a pharmaceutically acceptable carrier; the present invention also relates to a use of the compounds having the formula (I) or salts thereof in the preparation of a medicament as a 5-HT₄ receptor agonist. In other words, the present invention also provides the composition comprising the above compounds in an effective therapeutic amount, and the use of the compounds in the preparation of a medicament as a 5-HT₄ receptor agonist.

### SPECIFIC IMPLEMENTION METHODS

The following examples serve to illustrate the invention, but should not be considered as limiting the scope of the invention.

### EXAMPLES

The structures of compounds were confirmed by NMR and MS. NMR chemical shifts (δ) were given in part per million (ppm). NMR was performed on a Bruker AVANCE-400 spectrometer. The appropriate solvents were deuterated-methanol (CD₃OD) and deuterated-chloroform (CDCl₃) with tetramethylsilane (TMS) as internal standard and chemical shifts were given in 10⁻⁶ (ppm).

MS was determined by a FINNIGAN LCQAd (ESI) mass spectrometer (Therm, Finnigan LCQ advantage MAX).

Kinase average inhibition rate and IC₅₀ were determined by a NovoStar ELIASA (BMG Co., Germany).

The thin-layer silica gel was Yantai Huanghai HSGF254 or Qingdao GF254 silica gel plate.

Column chromatography generally used Yantai Huanghai 200∼300 mesh silica gel as carrier.
CD₃OD: deuterated-methanol;
CDCl₃: deuterated-chloroform.

### Example 1

### (R)-Quinuclidin-3-yl 5-((S)-2-((4-amino-5-chloro-2-ethoxybenzamido)methyl)morpholino)pentanoate

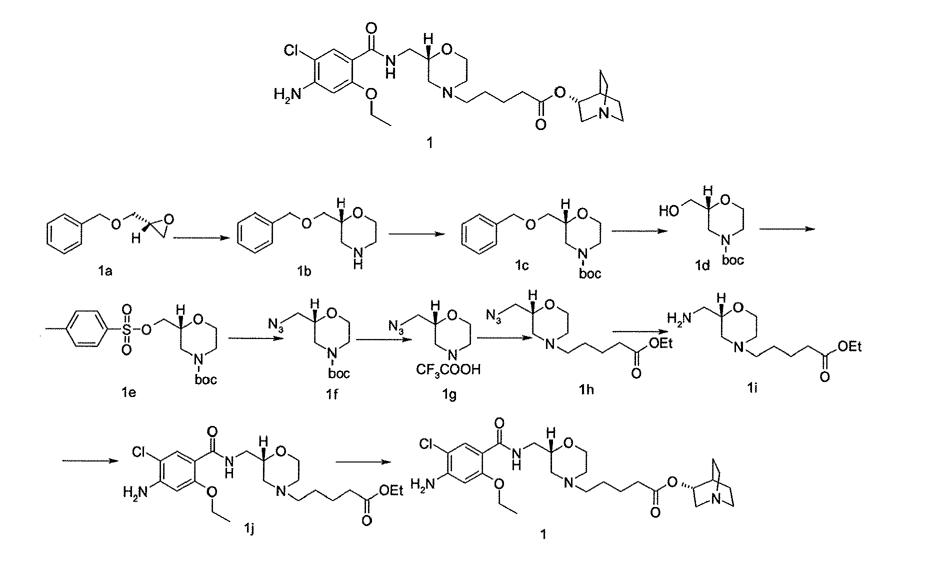

Mono-(2-amino-ethyl) sulfate (35.2 g, 250 mmol) was dissolved in 160 mL of aqueous sodium hydroxide solution (40%) with stirring, and to the mixture was added with a solution of (R)benzyl glycidyl ether **1a** (8.2 g, 50 mmol) in methanol. The reaction mixture was reacted overnight at 50°C and monitored by thin layer chromatography until the disappearance of the starting materials. The reaction mixture was diluted with 100 mL of water and 100 mL of concentrated hydrochloric acid, and extracted with dichloromethane (200 mL×3). The combined organic phase was washed successively with water (200 mL) and saturated brine (200 mL), dried over anhydrous magnesium sulfate, filtered and concentrated under reduced pressure. The residue was purified by silica gel column chromatography to obtain the title compound (R)-2-benzyloxymethyl-morpholine **1b** (5.08 g, yield 49%) as a yellow liquid.
MS m/z (ESI): 208.7 [M+1].
¹H NMR (CDCl₃, 400 MHz) δ 7.4-7.2 (m, 5H), 4.56 (s, 2H), 3.9 (d, 1H, J=11Hz) 3.8-3.35 (m, 4H), 2.92 (dd, 1H, J₁=2.5Hz, J₂=12.0Hz), 2.85-2.75 (m, 2H), 2.66 (dd, 1H, J₁=10.5Hz, J₂=12.0Hz), 2.4 (s, 1H).

(R)-2-Benzyloxymethyl-morpholine **1b** (2.07 g, 10 mmol) was dissolved in 20 mL of dichloromethane with stirring, and to the mixture was added potassium carbonate (1.38 g, 10 mmol) and di-tert-butyl-dicarbonate (3.27 g, 15 mmol). The reaction mixture was stirred overnight at room temperature and monitored by thin layer chromatography until the disappearance of the starting materials. The resulting mixture was added with saturated sodium bicarbonate solution (20 mL), and extracted with ethyl acetate (50 mL×3). The combined organic phase was washed successively with water (100 mL) and saturated brine (100 mL), dried over anhydrous magnesium sulfate, filtered and concentrated under reduced pressure. The residue was purified by silica gel column chromatography to obtain the title compound (R)-2-benzyloxymethyl-morpholine-4-carboxylic acid tert-butyl ester **1c** (1.967 g, yield 64%) as a colorless transparent liquid.
MS m/z (ESI): 308.1 [M+1].
¹H NMR (CDCl₃, 400 MHz) δ 7.35-7.25 (m, 5H), 4.56 (s, 2H), 3.91-3.88 (m, 3H), 3.53-3.45 (m, 4H), 3.0-2.9 (m, 1H), 2.8-2.7 (m, 1H), 1.46 (s, 9H).

(R)-2-Benzyloxymethyl-morpholine-4-carboxylic acid tert-butyl ester **1c** (1.02 g, 3.32 mmol) and 0.45 g of palladium on activated carbon were added to a reaction flask. The reaction flask was purged with hydrogen for three times. 10 mL of ethanol was added under hydrogen atmosphere. The reaction mixture was stirred overnight at 70°C and monitored by thin layer chromatography until the disappearance of the starting materials. The resulting mixture was purified by silica gel column chromatography to obtain the title compound (R)-2-hydroxymethyl-morpholine-4-carboxylate acid tert-butyl ester **1d** (0.428 g, yield 59%) as a colorless oil.
MS m/z (ESI): 240.3 [M+1].
¹H NMR (CDCl₃, 400 MHz) δ 3.92-3.88 (m, 3H), 3.7-3.64 (m, 1H), 3.60-3.48 (m, 3H), 2.936 (m, 1H), 2.75 (m, 1H), 2.06 (m, 1H), 1.46 (s, 9H).

(R)-2-Hydroxymethyl-morpholine-4-carboxylate acid tert-butyl ester **1d** (0.217 g, 1 mmol) was dissolved in 10 mL of dichloromethane with stirring in an ice-water bath, and added with p-toluenesulfonyl chloride (0.324 g, 1.7 mmol) and triethylamine (0.252 g, 2.5 mmol). The reaction mixture was stirred overnight at room temperature and monitored by thin layer chromatography until the disappearance of the starting materials. The resulting mixture was added with saturated sodium bicarbonate solution (20 mL), and extracted with ethyl acetate (50 mL×3). The combined organic phase was washed successively with water (50 mL) and saturated brine (50 mL), dried over anhydrous magnesium sulfate, filtered and concentrated under reduced pressure. The residue was purified by silica gel column chromatography to obtain the title compound (R)-2-(toluene-4-sulfonyloxymethyl)-morpholine-4-carboxylic acid tert-butyl ester **1e** (0.203 g, yield 55%) as a colorless viscous liquid.
MS m/z (ESI): 394.3 [M+23].
¹H NMR (CDCl₃, 400 MHz) δ 7.8-7.78 (d, 2H), 7.36-7.34 (d, 2H), 4.05-3.97 (m, 2H), 3.9-3.8 (m, 3H), 3.61-3.58 (m, 1H), 3.49-3.43 (m, 1H), 2.9-2.85 (m, 1H), 2.7-2.61 (m, 1H), 2.452 (s, 3H), 1.45 (s, 9H).

(R)-2-(Toluene-4-sulfonyloxymethyl)-morpholine-4-carboxylic acid tert-butyl ester **1e** (0.186 g, 0.501 mmol) was dissolved in 10 mL of N,N-dimethylformamide with stirring, and added with sodium azide (90 mg, 1.002 mmol). The reaction mixture was heated to 90°C, reacted for 3 hours and monitored by thin layer chromatography until the disappearance of the starting materials. The resulting mixture was added with saturated sodium bicarbonate solution (10 mL), and extracted with ethyl acetate (50 mL×3). The combined organic phase was washed successively with water (50 mL) and saturated brine (50 mL), dried over anhydrous magnesium sulfate, filtered and concentrated under reduced pressure. The residue was purified by silica gel column chromatography to obtain the title compound (R)-2-azidomethyl-morpholine-4-carboxylate acid tert-butyl ester **1f** (0.101 g, yield 83.4%) as a white snow-like solid.
MS m/z (ESI): 243.5 [M+1].
¹H NMR (CDCl₃, 400 MHz) δ 3.92-3.89 (dd, 1H, J₁=11.2 Hz, J₂=1.6Hz), 3.65-3.58 (m, 2H), 3.31-3.19 (m, 2H), 2.91-2.8 (m, 3H), 2.67-2.62 (m, 1H), 2.0-1.8 (br, 1H).

(R)-2-Azidomethyl-morpholine-4-carboxylate acid tert-butyl ester **1f** (0.65 g, 2.681 mmol) was dissolved in 30 mL of dichloromethane with stirring, and added with 3 mL of trifluoroacetic acid. The reaction mixture was stirred for 40 minutes and monitored by thin layer chromatography until the disappearance of the starting materials. The resulting mixture was concentrated to obtain the title compound (R)-2-azidomethyl-morpholine trifluoroacetate **1g** (0.318 g) for direct use in the next step.
¹H NMR (CDCl₃, 400 MHz) δ 3.92-3.89 (dd, 1H, J₁=11.2 Hz, J₂=1.6Hz), 3.65-3.58 (m, 2H), 3.31-3.19 (m, 2H), 2.91-2.8 (m, 3H), 2.67-2.62 (m, 1H), 2.0-1.8 (br, 1H).

(R)-2-Azidomethyl-morpholine trifluoroacetate **1g** (0.381 g, 2.68 mmol) was dissolved in 50 mL of acetonitrile with stirring, and added with potassium carbonate (1.11 g, 8.04 mmol). After stirring for 5 minutes, 5-bromo-pentanoic acid ethyl ester (673 mg, 3.217 mmol) was added. The reaction mixture was heated to reflux overnight and monitored by thin layer chromatography until the disappearance of the starting materials. The reaction mixture was quenched with water (10 mL) and extracted with ethyl acetate (100 mL×3). The combined organic phase was washed with saturated brine (200 mL), dried over anhydrous magnesium sulfate, filtered and concentrated under reduced pressure. The residue was purified by silica gel column chromatography to obtain the title compound (R)-6-(2-azidomethyl-morpholin-4-yl)-pentanoic acid ethyl ester **1h** (676 mg, yield 93.3%) as a light yellow transparent oil.
MS m/z (ESI): 271.1 [M+1].
¹H NMR (CDCl₃, 400 MHz) δ 4.14-4.12 (m, 2H), 3.93-3.90 (d, 1H, J=11.6Hz), 3.71-3.68 (m, 2H), 3.32-3.28 (m, 2H), 2.75-2.69 (m, 2H), 2.38-2.3 (m, 4H), 2.16-2.10 (t, 1H, J=11.2Hz), 1.94-1.89 (t, 1H, J=10.8Hz), 1.68-1.64 (m, 2H), 1.54-1.51 (m, 2H), 1.28-1.24 (t, 3H, J=7.2Hz).

(R)-6-(2-Azidomethyl-morpholin-4-yl)-pentanoic acid ethyl ester **1h** (338 mg, 1.25 mmol) was dissolved in 50 mL of ethanol with stirring, and added with 40.0 mg of palladium on activated carbon. The reaction mixture was purged with hydrogen for three times, reacted overnight under hydrogen atmosphere and monitored by thin layer chromatography until the disappearance of the starting materials. The resulting mixture was filtered under reduced pressure and concentrated to obtain the title compound (S)-6-(2-aminomethyl-morpholin-4-yl)-pentanoic acid ethyl ester **1i** (0.18 g) as a light yellow oil for direct use in the next step.
MS m/z (ESI): 259.8 [M+1].

4-Amino-5-chloro-2-ethoxy-benzoic acid (0.158 g, 0.737 mmol) was dissolved in 20 mL of dichloromethane with stirring, and added with (S)-6-(2-aminomethyl-morpholin-4-yl)-pentanoic acid ethyl ester **1i** (0.18 g, 0.737 mmol) and 1-ethyl-3-(3-dimethyl-propyl)carbodiimide hydrochloride (0.14 g, 0.737 mmol). The reaction mixture was stirred at room temperature for 5 hours and monitored by thin layer chromatography until the disappearance of the starting materials. The resulting mixture was added with 100 mL of saturated sodium bicarbonate solution, and extracted with ethyl acetate (200 mL×3). The combined organic phase was washed successively with water (100 mL) and saturated brine (100 mL), dried over anhydrous magnesium sulfate, filtered and concentrated under reduced pressure. The residue was purified by silica gel column chromatography to obtain the title compound (S)-5-{2-[(4-amino-5-chloro-2-ethoxy-benzoylamino)-methyl]-morpholin-4-yl}-pentanoic acid ethyl ester **1j** (0.2 g, yield 63.5%) as a white solid.
MS m/z (ESI): 442.3 [M+1].

(S)-5-{2-[(4-Amino-5-chloro-2-ethoxy-benzoylamino)-methyl]-morpholin-4-yl}-pentanoic acid ethyl ester **1j** (0.207 g, 0.468 mmol) was dissolved in 30 mL of toluene with stirring, and added with 1-aza-bicyclo[2.2.2]octan-3-ol (0.357g, 2.81 mmol). Tetraethyl titanate (0.16 g, 0.702 mmol) was added at room temperature. The reaction mixture was heated to reflux overnight and monitored by thin layer chromatography until the disappearance of the starting materials. The resulting mixture was added with 40 mL of ethyl acetate, and washed with saturated sodium bicarbonate solution (100 mL) and saturated brine (100 mL), dried over anhydrous magnesium sulfate, filtered and concentrated under reduced pressure. The residue was purified by silica gel column chromatography to obtain the title compound (R)-quinuclidin-3-yl 5-((S)-2-((4-amino-5-chloro-2-ethoxybenzamido)methyl)morpholino)pentanoate **1** (57 mg, yield 23.6%) as a white solid.
MS m/z (ESI): 523.4 [M+1].
¹H NMR (CDCl₃, 400 MHz) δ 8.22(s, 1H), 8.11 (s, 1H), 6.27 (s, 1H), 4.87-4.85 (m, 1H), 4.32 (s, 2H), 4.12-4.06 (q, 2H), 3.87-3.86 (m, 1H), 3.71-3.65 (m, 1H), 3.38-3.29 (m, 1H), 3.0-2.26 (m, 1H), 2.14-2.10 (m, 2H), 1.99-1.89 (m, 1H), 1.57-1.47 (t, 3H).

### Example 2

### (R)-Quinuclidin-3-yl 5-((S)-2-((4-amino-5-chloro-2-ethoxybenzamido)methyl)morpholino)pentanoate

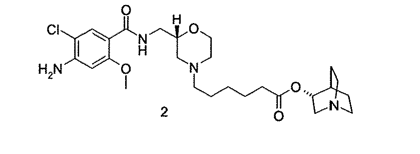

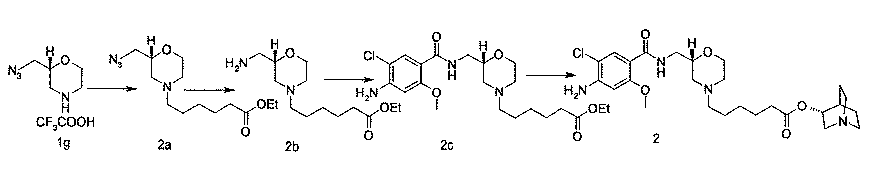

(R)-2-Azidomethyl-morpholine trifluoroacetate **1g** (0.803 g, 1.88 mmol) was dissolved in 40 mL of acetonitrile with stirring, and added with potassium carbonate (0.78 g, 5.64 mmol). After stirring for 5 minutes, 6-bromo-hexanoic acid ethyl ester (0.503 g, 2.258 mmol) was added. The reaction mixture was heated to reflux overnight and monitored by thin layer chromatography until the disappearance of the starting materials. The resulting mixture was concentrated and 300 mL of dichloromethane was added to the residue, the resulting mixture was washed with saturated brine (100 mL), dried over anhydrous magnesium sulfate, filtered and concentrated under reduced pressure. The residue was purified by silica gel column chromatography to obtain the title compound (R)-6-(2-azidomethyl-morpholin-4-yl)-hexanoic acid ethyl ester **2a** (404 mg, yield 75.5%) as a light yellow transparent liquid.
MS m/z (ESI): 285.1 [M+1].
¹H NMR (CDCl₃, 400 MHz) δ 4.15-4.09 (q, 2H), 3.92-3.88 (m, 1H), 3.71-3.63 (m, 2H), 3.34-3.24 (m, 2H), 2.74-2.67 (m, 2H), 2.35-2.28 (m, 4H), 2.15-2.09 (m, 1H), 1.9 (t, 1H), 1.65-1.55 (m, 2H), 1.55-1.45 (m, 2H), 1.45-1.3 (m, 2H), 1.3-1.2 (t, 3H).

(R)-6-(2-Azidomethyl-morpholin-4-yl)-hexanoic acid ethyl ester **2a** (1.3 g, 4.572 mmol) was dissolved in 50 mL of ethanol with stirring, and added with 0.13 g of palladium on activated carbon. The reaction mixture was purged with hydrogen for three times, reacted for 2.5 hours under hydrogen atmosphere and monitored by thin layer chromatography until the disappearance of the starting materials. The resulting mixture was filtered and concentrated to obtain the title compound (S)-6-(2-aminomethyl-morpholin-4-yl)-hexanoic acid ethyl ester **2b** for direct use in the next step.
MS m/z (ESI): 259.8 [M+1].

4-Amino-5-chloro-2-methoxy-benzoic acid (0.277 g, 1.37 mmol) was dissolved in 20 mL of dichloromethane with stirring, and added with (S)-6-(2-aminomethyl-morpholin-4-yl)-hexanoic acid ethyl ester **2b** (0.294 g, 1.14 mmol) and 1-ethyl-3-(3-dimethyl-propyl)carbodiimide hydrochloride (0.437 g, 2.28 mmol). The reaction mixture was reacted overnight at room temperature and monitored by thin layer chromatography until the disappearance of the starting materials. 400 mL of dichloromethane was added. The mixture was washed successively with saturated sodium bicarbonate solution (100 mL) and saturated brine (100 mL), dried over anhydrous magnesium sulfate, filtered and concentrated under reduced pressure. The residue was purified by silica gel column chromatography to obtain the title compound (S)-5-{2-[(4-amino-5-chloro-2-methoxy-benzoylamino)-methyl]-morpholin-4-yl}-hexanoic acid ethyl ester **2c** (0.119 g, yield 23.6%) as a white solid.
MS m/z (ESI): 442.3 [M+1].
¹H NMR (CDCl₃, 400 MHz) δ 8.09 (s, 1H), 7.9 (m, 1H), 6.28 (s, 1H), 4.4 (s, 2H), 4.14-4.09 (q, 2H), 3.88 (s, 3H), 3.88 (m, 1H), 3.71-3.65 (m, 3H), 3.39-3.35 (m, 1H), 2.81-2.78 (m, 1H), 2.67 (m, 1H), 2.33-2.27 (m, 4H), 2.09-2.08 (m, 1H), 1.91-1.86 (m, 1H), 1.65-1.61 (m, 2H), 1.49-1.47 (m, 2H), 1.34-1.33 (m, 2H), 1.26-1.23 (t, 3H).

(S)-5-{2-[(4-Amino-5-chloro-2-methoxy-benzoylamino)-methyl]-morpholin-4-yl}-hexanoic acid ethyl ester **2c** (0.119 g, 0.269 mmol) was dissolved in 40 mL of toluene with stirring, and added with 1-aza-bicyclo[2.2.2]octan-3-ol (0.171 g, 1.347 mmol). Tetraethyl titanate (92 mg, 0.404 mmol) was added at room temperature. The reaction mixture was heated to reflux overnight and monitored by thin layer chromatography until the disappearance of the starting materials. The resulting mixture was diluted with 40 mL of water, and extracted with ethyl acetate (100 mL×3). The combined organic phase was washed with saturated sodium bicarbonate solution (100 mL) and saturated brine (100 mL), dried over anhydrous magnesium sulfate, filtered and concentrated under reduced pressure. The residue was purified by silica gel column chromatography to obtain the title compound (R)-quinuclidin-3-yl 5-((S)-2-((4-amino-5-chloro-2-ethoxybenzamido)methyl)morpholino)pentanoate **2** (13 mg, yield 10%) as a white solid.
MS m/z (ESI): 442.3 [M+1].
¹H NMR (CDCl₃, 400 MHz) δ 8.088 (s, 1H), 7.98 (s, 1H), 6.29 (s, 1H), 4.81-4.79 (m, 1H), 4.42 (s, 2H), 3.90-3.88 (s, 3H), 3.90-3.88 (m, 1H), 3.70-3.66 (m, 3H), 3.37-3.34 (m, 1H), 3.27-3.22 (m, 1H), 2.92-2.86 (m, 7H), 2.84-2.75 (m, 3H), 2.70-2.67 (m, 2H), 2.34-2.3 (m, 4H), 2.12-2.07 (m, 1H), 2.01 (m, 1H), 1.88-1.83 (m, 2H), 1.70-1.3 (m, 9H).

### Example 3

### (R)-Quinuclidin-3-yl 5-((S)-2-((4-amino-5-chloro-2-ethoxybenzamido)methyl)morpholino)hexanoate

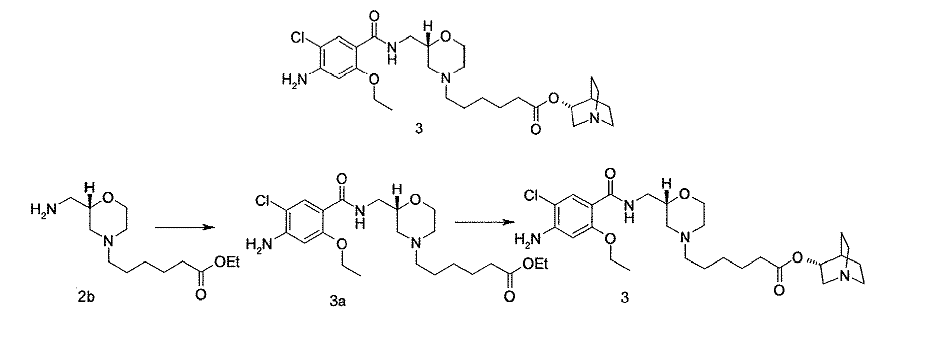

4-Amino-5-chloro-2-ethoxy-benzoic acid (0.295 g, 1.37 mmol) was dissolved in 20 mL of dichloromethane with stirring, and added with (S)-6-(2-aminomethyl-morpholin-4-yl)-hexanoic acid ethyl ester **2b** (0.294 g, 1.14 mmol) and 1-ethyl-3-(3-dimethyl-propyl)carbodiimide hydrochloride (0.437 g, 2.28 mmol). The reaction mixture was reacted overnight at room temperature and monitored by thin layer chromatography until the disappearance of the starting materials. The reaction mixture was diluted with 400 mL of dichloromethane, washed successively with saturated sodium bicarbonate solution (100 mL) and saturated brine (100 mL), dried over anhydrous magnesium sulfate, filtered and concentrated under reduced pressure. The residue was purified by silica gel column chromatography to obtain the title compound (S)-5-{2-[(4-amino-5-chloro-2-ethoxy-benzoylamino)-methyl]-morpholin-4-yl}-hexanoic acid ethyl ester **3a** (0.191 g, yield 36.8%) as a white solid.
MS m/z (ESI): 456.2 [M+1].
¹H NMR (CDCl₃, 400 MHz) δ 8.2 (m, 1H), 8.10 (s, 1H), 6.28 (s, 1H), 4.4 (s, 2H), 4.18-4.06 (m, 4H), 3.9-3.88 (m, 1H), 3.71-3.66 (m, 4H), 3.37-3.34 (m, 1H), 2.81-2.78 (d, 1H, J=11.2Hz), 2.71-2.68 (d, 1H, J=11.6Hz), 2.34-2.27 (m, 4H), 2.15-2.09 (m, 1H), 1.94-1.89 (m, 1H), 1.67-1.6 (m, 2H), 1.51-1.46 (m, 5H), 1.37-1.31 (m, 2H), 1.27-1.23 (m, 3H).

(S)-5-{2-[(4-Amino-5-chloro-2-ethoxy-benzoylamino)-methyl]-morpholin-4-yl}-hexanoic acid ethyl ester **3a** (0.191 g, 0.419 mmol) was dissolved in 40 mL of toluene with stirring, and added with 1-aza-bicyclo[2.2.2]octan-3-ol (0.266 g, 2.095 mmol). Tetraethyl titanate (143 mg, 0.628 mmol) was added at room temperature. The reaction mixture was heated to reflux overnight and monitored by thin layer chromatography until the disappearance of the starting materials. The resulting mixture was diluted with 300 mL of dichloromethane, washed with saturated sodium bicarbonate solution (100 mL) and saturated brine (100 mL), dried over anhydrous magnesium sulfate, filtered and concentrated under reduced pressure. The residue was purified by silica gel column chromatography to obtain the title compound (R)-quinuclidin-3-yl 5-((S)-2-((4-amino-5-chloro-2-ethoxybenzamido)methyl)morpholino)hexanoate **3** (20 mg, yield 9.13%) as a white solid.
MS m/z (ESI): 537.4 [M+1].
¹H NMR (CDCl₃, 400 MHz) δ 8.2 (s, 1H), 8.10 (s, 1H), 6.27 (s, 1H), 4.8 (m, 1H), 4.40 (s, 2H), 4.1-4.05 (q, 2H, J=6.8Hz), 3.88 (d, 1H,J=6.4Hz), 3.71-3.66 (m, 3H), 3.37-3.31 (m, 1H), 3.28-3.22 (m, 1H), 2.92-2.68 (m, 7H), 2.34-2.3 (m, 4H), 2.15-2.08 (m, 1H), 2.02-2.01 (m, 1H), 1.93-1.86 (m, 2H), 1.74-1.69 (m, 3H), 1.49-1.47 (m, 3H), 1.49-1.47 (t, 3H, J=6.8Hz), 1.4-1.3 (m, 3H).

### Example 4

### (R)-Quinuclidin-3-yl 5-((R)-2-((4-amino-5-chloro-2-methoxybenzamido)methyl)morpholino)hexanoate

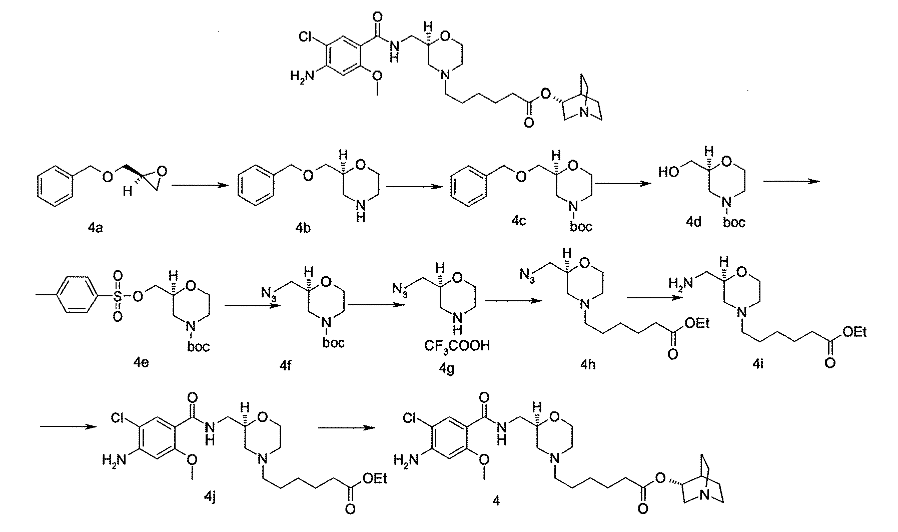

Mono-(2-amino-ethyl) sulfate (35.2 g, 250 mmol) was dissolved in 60 mL of aqueous sodium hydroxide solution (40%) with stirring. The solution of (S)-benzyl glycidyl ether **4a** (8.2 g, 50 mmol) in methanol was added to the above sodium hydroxide solution. After reacting for 1 hour at 50°C, 100 mL of aqueous sodium hydroxide solution (40%) was added. The reaction mixture was stirred at 50-55°C for 16 hours and monitored by thin layer chromatography until the disappearance of the starting materials. The resulting mixture was diluted with 100 mL of water and 100 mL of concentrated hydrochloric acid was added dropwise. The mixture was extracted with dichloromethane (500 mL×4). The combined organic phase was washed successively with water (300 mL) and saturated brine (300 mL), dried over anhydrous magnesium sulfate, filtered and concentrated under reduced pressure. The residue was purified by silica gel column chromatography to obtain the title compound (S)-2-benzyloxymethyl-morpholine **4b** (5.08 g, yield 49%) as a yellow liquid.
MS m/z (ESI): 208.7[M+1].
¹H NMR (CDCl₃, 400 MHz) δ 7.4-7.2(m, 5H), 4.56 (s, 2H), 3.9 (d, 1H, J=11Hz), 3.8-3.35 (m, 4H), 2.92 (dd, 1H, J₁=2.5Hz, J₂=12.0Hz), 2.85-2.75 (m, 2H), 2.66 (dd, 1H, J₁=10.5Hz, J₂=12.0Hz), 2.4 (s, 1H).

(S)-2-Benzyloxymethyl-morpholine **4b** (2.07 g, 10 mmol) was dissolved in 20 mL of dichloromethane with stirring, and added with potassium carbonate (1.38 g, 10 mmol) and di-tert-butyl dicarbonate (3.27 g, 15 mmol). The reaction mixture was stirred overnight at room temperature and monitored by thin layer chromatography until the disappearance of the starting materials. The resulting mixture was quenched with saturated sodium bicarbonate solution (50 mL), and extracted with ethyl acetate (200 mL×3). The combined organic phase was washed successively with water (50 mL) and saturated brine (50 mL), dried over anhydrous magnesium sulfate, filtered and concentrated under reduced pressure. The residue was purified by silica gel column chromatography to obtain the title compound (S)-2-benzyloxymethyl-morpholine-4-carboxylic acid tert-butyl ester **4c** (1.967 g, **yield** 64%) as a colorless transparent liquid.
MS m/z (ESI): 308.1[M+1].
¹H NMR (CDCl₃, 400 MHz) δ 7.35-7.25 (m, 5H), 4.56 (s, 2H), 3.91-3.88 (m, 3H), 3.53-3.45 (m, 4H), 3.0-2.9 (m, 1H), 2.8-2.7 (m, 1H), 1.46 (s, 9H).

(S)-2-Benzyloxymethyl-morpholine-4-carboxylic acid tert-butyl ester **4c** (5.27 g, 17.2 mmol) was dissolved in 200 mL of ethanol with stirring, and added with 0.527 g of palladium on activated carbon and two drops of 1N hydrochloric acid. The reaction mixture was purged with hydrogen for three times, reacted at 70°C overnight under hydrogen atmosphere and monitored by thin layer chromatography until the disappearance of the starting materials. The resulting mixture was quenched with saturated sodium bicarbonate solution (100 mL) and extracted with ethyl acetate (200 mL×4). The combined organic phase was washed successively with water (100 mL) and saturated brine (100 mL), dried over anhydrous magnesium sulfate, filtered and concentrated under reduced pressure. The residue was purified by silica gel column chromatography to obtain the title compound (S)-2-hydroxymethyl-morpholine-4-carboxylic acid tert-butyl ester **4d** (3.646 g, yield 97.7%) as a colorless or light yellow liquid.
MS m/z (ESI): 240.3 [M+23].
¹H NMR (CDCl₃, 400 MHz) δ 3.92-3.88 (m, 3H), 3.7-3.64 (m, 1H), 3.60-3.48 (m, 3H), 2.936 (m, 1H), 2.75 (m, 1H), 2.06 (m, 1H), 1.46 (s, 9H).

(S)-2-Hydroxymethyl-morpholine-4-carboxylic acid tert-butyl ester **4d** (0.217 g, 1 mmol) was dissolved in 10 mL of dichloromethane with stirring, and added with p-toluenesulfonyl chloride (0.324 g, 1.7 mmol) and triethylamine (0.252 g, 2.5 mmol) in an ice-water bath. The reaction mixture was reacted overnight at room temperature and monitored by thin layer chromatography until the disappearance of the starting materials. The resulting mixture was quenched with saturated sodium bicarbonate solution (10 mL), and extracted with dichloromethane (50 mL×3). The combined organic phase was washed successively with water (50 mL) and saturated brine (50 mL), dried over anhydrous magnesium sulfate, filtered and concentrated under reduced pressure. The residue was purified by silica gel column chromatography to obtain the title compound (S)-2-(toluene-4-sulfonyloxymethyl)-morpholine-4-carboxylic acid tert-butyl ester **4e** (0.203 g, yield 55%) as a colorless viscous liquid.
MS m/z (ESI): 394.3 [M+23].
¹H NMR (CDCl₃, 400 MHz) δ 7.8-7.78 (d, 2H), 7.36-7.34 (d, 2H), 4.05-3.97 (m, 2H), 3.9-3.8 (m, 3H), 3.61-3.58 (m, 1H), 3.49-3.43 (m, 1H), 2.9-2.85 (m, 1H), 2.7-2.61 (m, 1H), 2.452 (s, 3H), 1.45 (s, 9H).

(S)-2-(Toluene-4-sulfonyloxymethyl)-morpholine-4-carboxylic acid tert-butyl ester **4e** (0.186 g, 0.501 mmol) was dissolved in 10 mL of N,N-dimethylformamide with stirring, and added with sodium azide (90 mg, 1.002 mmol). The reaction mixture was heated to 90°C, reacted for 3 hours and monitored by thin layer chromatography until the disappearance of the starting materials. The resulting mixture was quenched with saturated sodium bicarbonate solution (20 mL) and extracted with ethyl acetate (50 mL×3). The combined organic phase was washed successively with water (100 mL) and saturated brine (100 mL), dried over anhydrous magnesium sulfate, filtered and concentrated under reduced pressure. The residue was purified by silica gel column chromatography to obtain the title compound (S)-2-azidomethyl-morpholine-4-carboxylic acid tert-butyl ester **4f** (0.101 g, yield 83.4%) as a colorless oil.
MS m/z (ESI): 243.5 [M+1].
¹H NMR (CDCl₃, 400 MHz) δ 3.95-3.8 (m, 3H), 3.6-3.5 (m, 2H), 3.31-3.3 (m, 2H), 2.94 (m, 1H), 2.72 (m, 1H), 1.46 (s, 9H).

(S)-2-Azidomethyl-morpholine-4-carboxylic acid tert-butyl ester **4f** (91 mg, 0.376 mmol) was dissolved in 5 mL of dichloromethane with stirring, and added with 0.5 mL of trifluoroacetic acid. The reaction mixture was stirred for 10 minutes and monitored by thin layer chromatography until the disappearance of the starting materials. The mixture was concentrated to obtain the title compound (S)-2-azidomethyl-morpholine trifluoroacetate **4g** for direct use in the next step.
¹H NMR (CDCl₃, 400 MHz) δ 3.92-3.89 (dd, 1H, J₁=11.2 Hz, J₂=1.6Hz), 3.65-3.58 (m, 2H), 3.31-3.19 (m, 2H), 2.91-2.8 (m, 3H), 2.67-2.62 (m, 1H), 2.0-1.8 (br, 1H).

(S)-2-Azidomethyl-morpholine trifluoroacetate **4g** (2.96 g, 11.56 mmol) was dissolved in 50 mL of acetonitrile with stirring, and added with potassium carbonate (4.79 g, 34.68 mmol). After stirring for 10 minutes, 6-bromo-hexanoic acid ethyl ester (2.84 g, 12.72 mmol) was added. The reaction mixture was heated to reflux for 1.5-2 hours and monitored by thin layer chromatography until the disappearance of the starting materials. The reaction mixture was quenched with saturated sodium bicarbonate solution (100 mL) and extracted with dichloromethane (300 mL×4). The combined organic phase was washed with saturated brine (200 mL), dried over anhydrous magnesium sulfate, filtered and concentrated under reduced pressure. The residue was purified by silica gel column chromatography to obtain the title compound (S)-6-(2-azidomethyl-morpholin-4-yl)-hexanoic acid ethyl ester **4h** (2.914 g, yield 88.8%) as a colorless or light yellow transparent liquid.
MS m/z (ESI): 285.2 [M+1].
¹H NMR (CDCl₃, 400 MHz) δ 4.15-4.09 (q, 2H), 3.92-3.88 (m, 1H), 3.71-3.63 (m, 2H), 3.34-3.24 (m, 2H), 2.74-2.67 (m, 2H), 2.35-2.28 (m, 4H), 2.15-2.09 (m, 1H), 1.9 (t, 1H), 1.65-1.55 (m, 2H), 1.55-1.45 (m, 2H), 1.45-1.3 (m, 2H), 1.3-1.2 (t, 3H).

(S)-6-(2-Azidomethyl-morpholin-4-yl)-hexanoic acid ethyl ester **4h** (87 mg, 0.306 mmol) was dissolved in 10 mL of methanol with stirring, and added with 9.0 mg of palladium on activated carbon. The reaction mixture was purged with hydrogen for three times, reacted under a hydrogen atmosphere overnight and monitored by thin layer chromatography until the disappearance of the starting materials. The mixture was filtered and concentrated to obtain the title compound (R)-6-(2-aminomethyl-morpholin-4-yl)-hexanoic acid ethyl ester **4i** for direct use in the next step.
MS m/z (ESI): 259.8 [M+1].

4-Amino-5-chloro-2-methoxy-benzoic acid (0.484 g, 2.4 mmol) was dissolved in 20 mL of dichloromethane with stirring, and added with (R)-6-(2-aminomethyl-morpholin-4-yl)-hexanoic acid ethyl ester **4i** (0.516 g, 2.0 mmol) and 1-ethyl-3-(3-dimethyl-propyl)carbodiimide hydrochloride (0.767 g, 4.0 mmol). The reaction mixture was reacted for 5 hours at room temperature and monitored by thin layer chromatography until the disappearance of the starting materials. The reaction mixture was added with 100 mL of saturated sodium bicarbonate solution, and extracted with dichloromethane (200 mL×3). The combined organic phase was washed successively with water and saturated brine (100 mL), dried over anhydrous magnesium sulfate, filtered and concentrated under reduced pressure. The residue was purified by silica gel column chromatography to obtain the title compound (R)-5-{2-[(4-amino-5-chloro-2-methoxy-benzoylamino)-methyl]-morpholin-4-yl}-hexanoic acid ethyl ester **4j** (153 mg, yield 17.3%) as a white solid.
MS m/z (ESI): 442.7 [M+1].
¹H NMR (CDCl₃, 400 MHz) δ 8.09 (s, 1H), 7.9 (m, 1H), 6.28 (s, 1H), 4.4 (s, 2H), 4.14-4.09 (q, 2H), 3.88 (s, 3H), 3.88 (m, 1H), 3.71-3.65 (m, 3H), 3.39-3.35 (m, 1H), 2.81-2.78 (m, 1H), 2.67 (m, 1H), 2.33-2.27 (m, 4H), 2.09-2.08 (m, 1H), 1.91-1.86 (m, 1H), 1.65-1.61 (m, 2H), 1.49-1.47 (m, 2H), 1.34-1.33 (m, 2H), 1.26-1.23 (t, 3H).

(R)-5-{2-[(4-Amino-5-chloro-2-methoxy-benzoylamino)-methyl]-morpholin-4-yl}-hexanoic acid ethyl ester **4j** (0.125 g, 0.283 mmol) was dissolved in 30 mL of toluene with stirring, and added with 1-aza-bicyclo[2.2.2]octan-3-ol (0.18 g, 1.415 mmol). Tetraethyl titanate (97 mg, 0.424 mmol) was added at room temperature. The reaction mixture was heated to reflux overnight and monitored by thin layer chromatography until the disappearance of the starting materials. The reaction mixture was concentrated under reduced pressure to remove toluene. The residue was purified by silica gel column chromatography to obtain the title compound (R)-quinuclidin-3-yl
5-((R)-2-((4-amino-5-chloro-2-methoxybenzamido)methyl)morpholino)hexanoate **4** (35 mg, yield 23.6%) as a white solid.
MS m/z (ESI): 523.7 [M+1].
¹H NMR (CDCl₃, 400 MHz) δ 8.093 (s, 1H), 7.984 (s, 1H), 6.29 (s, 1H), 4.8-4.78 (m, 1H), 4.39 (s, 2H), 3.90-3.88 (s, 3H), 3.90-3.88 (m, 1H), 3.71-3.65 (m, 3H), 3.37-3.34 (m, 1H), 3.26-3.21 (m, 1H), 2.91-2.65 (m, 7H), 2.34-2.3 (m, 4H), 2.12-2.07 (m, 1H), 2.00-1.99 (m, 1H), 1.91-1.82 (m, 2H), 1.69-1.3 (m, 9H).

### Example 5 (R)-Quinuclidin-3-yl 5-((R)-2-((4-amino-5-chloro-2-ethoxybenzamido)methyl)morpholino)pentanoate

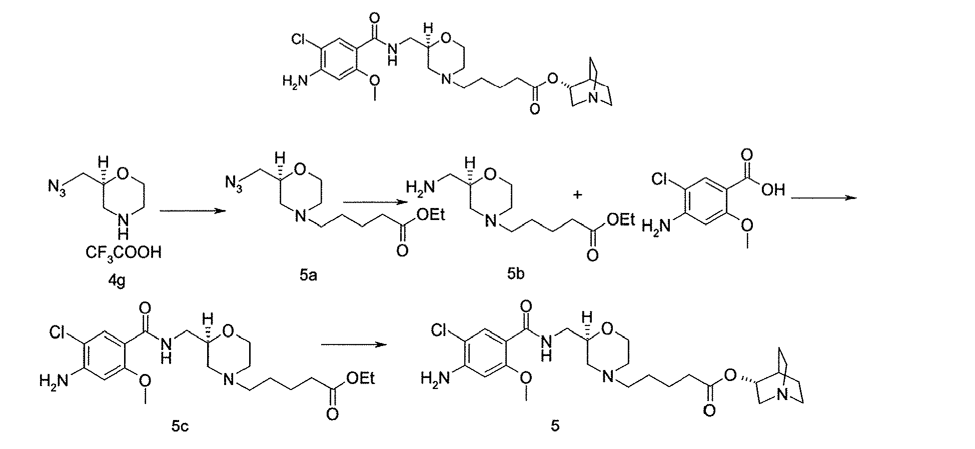

(S)-2-Azidomethyl-morpholine trifluoroacetate **4g** (0.64 g, 2.5 mmol) and potassium carbonate (1.037 g, 7.5 mmol) were dissolved in 30 mL of acetonitrile with stirring, and added with 5-bromo-pentanoic acid ethyl ester (0.575 g, 2.75 mmol). The reaction mixture was heated to reflux for 3 hours and monitored by thin layer chromatography until the disappearance of the starting materials. The resulting mixture was quenched with saturated sodium bicarbonate solution (50 mL) and extracted with ethyl acetate (100 mL×3). The combined organic phase was washed successively with water (100 mL) and saturated brine (100 mL), dried over anhydrous magnesium sulfate, filtered and concentrated under reduced pressure. The residue was purified by silica gel column chromatography to obtain the title compound (S)-6-(2-azidomethyl-morpholin-4-yl)-pentanoic acid ethyl ester **5a** (0.532 g, yield 78.8%) as a light yellow transparent liquid.
MS m/z (ESI): 271.1 [M+1].
¹H NMR (CDCl₃, 400 MHz) δ 4.14-4.12 (m, 2H), 3.93-3.90 (d, 1H, J=11.6Hz), 3.71-3.68 (m, 2H), 3.32-3.28 (m, 2H), 2.75-2.69 (m, 2H), 2.38-2.3 (m, 4H), 2.16-2.10 (t, 1H, J=11.2Hz), 1.94-1.89 (t, 1H, J=10.8Hz), 1.68-1.64 (m, 2H), 1.54-1.51 (m, 2H), 1.28-1.24 (t, 3H, J=7.2Hz).

(S)-6-(2-Azidomethyl-morpholin-4-yl)-pentanoic acid ethyl ester **5a** (532 mg, 1.97 mmol) and 53 mg of palladium on activated carbon were added to a reaction flask, the reaction mixture was purged with hydrogen for three times and was added with 20 mL of methanol with stirring. The reaction mixture was reacted for 4 hours under hydrogen atmosphere and monitored by thin layer chromatography until the disappearance of the starting materials. The resulting mixture was filtered and concentrated to obtain the title compound (R)-6-(2-aminomethyl-morpholin-4-yl)-pentanoic acid ethyl ester **5b** as a light yellow liquid for direct use in the next step.
MS m/z (ESI): 245.2 [M+1].

(R)-6-(2-Aminomethyl-morpholin-4-yl)-pentanoic acid ethyl ester **5b** (0.241 g, 1.0 mmol) was dissolved in 20 mL of dichloromethane with stirring, and added with 4-amino-5-chloro-2-methoxy-benzoic acid (0.242 g, 1.2 mmol) and 1-ethyl-3-(3-dimethyl-propyl)carbodiimide hydrochloride (0.383 g, 2.0 mmol). The reaction mixture was stirred for 3 hours at room temperature and monitored by thin layer chromatography until the disappearance of the starting materials. The reaction mixture was added with 100 mL of saturated sodium bicarbonate solution, and extracted with ethyl acetate (200 mL×3). The combined organic phase was washed with saturated brine (100 mL), dried over anhydrous magnesium sulfate, filtered and concentrated under reduced pressure. The residue was purified by silica gel column chromatography to obtain the title compound (R)-5-{2-[(4-amino-5-chloro-2-methoxy-benzoylamino)-methyl]-morpholin-4-yl}-pentanoic acid ethyl ester **5c** (162 mg, yield 45%) as a white solid.
MS m/z (ESI): 428.3 [M+1].

(R)-5-{2-[(4-Amino-5-chloro-2-methoxy-benzoylamino)-methyl]-morpholin-4-yl}-pentanoic acid ethyl ester 5c (0.16 g, 0.37 mmol) was dissolved in 20 mL of toluene with stirring, and added with 1-aza-bicyclo[2.2.2]octan-3-ol (0.235 g, 1.85 mmol). Tetraethyl titanate (0.127 g, 0.555 mmol) was added dropwise at room temperature. The reaction mixture was heated to reflux overnight and monitored by thin layer chromatography until the disappearance of the starting materials. The reaction mixture was concentrated to remove toluene, added with dichloromethane (50 mL) and saturated sodium bicarbonate solution (50 mL), and extracted with dichloromethane (100 mL×3). The combined organic phase was washed with saturated brine (200 mL), dried over anhydrous magnesium sulfate, filtered and concentrated under reduced pressure. The residue was purified by silica gel column chromatography to obtain the title compound (R)-quinuclidin-3-yl 5-((R)-2-((4-amino-5-chloro-2-methoxybenzamido)methyl)morpholino)pentanoate **5** (32 mg, yield 17%) as a light yellow solid.
MS m/z (ESI): 509.3 [M+1].
¹H NMR (CDCl₃, 400 MHz) δ 8.08 (s, 1H), 7.98 (s, 1H), 6.3 (s, 1H), 4.85-4.83 (m, 1H), 4.41 (s, 2H), 3.90-3.88 (s, 3H), 3.90-3.88 (m, 1H), 3.70-3.65 (m, 3H), 3.33 (m, 2H), 2.96-2.67 (m, 7H), 2.36-2.32 (m, 4H), 2.10-2.07 (m, 2H), 1.91-1.86 (m, 2H), 1.80-1.78 (m, 1H), 1.66-1.61 (m, 3H), 1.53-1.48 (m, 3H).

### Example 6

### (R)-Quinuclidin-3-yl 5-((R)-2-((4-amino-5-chloro-2-ethoxybenzamido)methyl)morpholino)butanoate

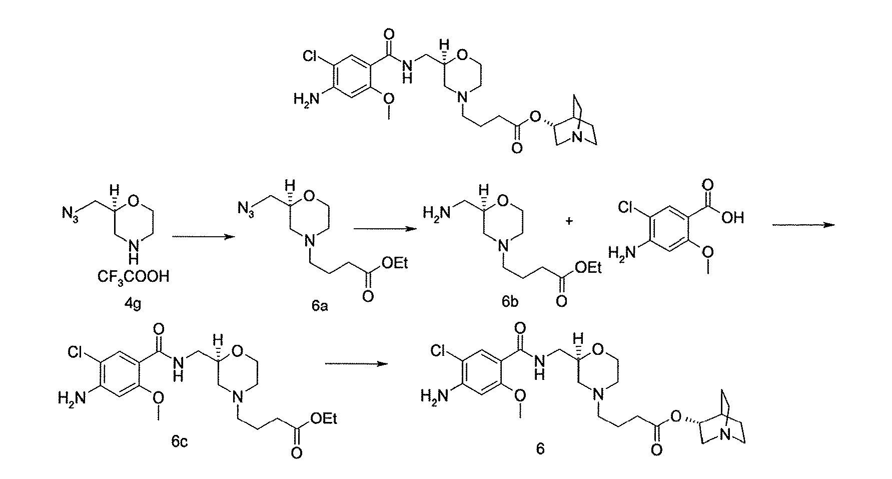

(S)-2-Azidomethyl-morpholine trifluoroacetate **4g** (0.64 g, 2.5 mmol) and potassium carbonate (1.037 g, 7.5 mmol) were dissolved in 30 mL of acetonitrile with stirring, and added with 4-bromo-butyric acid ethyl ester (0.585 g, 3.0 mmol). The reaction mixture was heated to reflux for 3 hours and monitored by thin layer chromatography until the disappearance of the starting materials. The reaction mixture was quenched with saturated sodium bicarbonate solution (50 mL), and extracted with ethyl acetate (50 mL×3). The combined organic phase was washed successively with water (100 mL) and saturated brine (100 mL), dried over anhydrous magnesium sulfate, filtered and concentrated under reduced pressure. The residue was purified by silica gel column chromatography to obtain the title compound (S)-6-(2-azidomethyl-morpholin-4-yl)-butyric acid ethyl ester **6a** (0.461 g, yield 72%) as a light yellow transparent liquid.
MS m/z (ESI): 257.1 [M+1].
¹H NMR (CDCl₃, 400 MHz) δ 4.17-4.10 (m, 2H, J=7.2Hz), 3.91-3.88 (m, 1H), 3.70-3.61 (m, 2H), 3.34-3.22 (m, 2H), 2.73-2.66 (m, 2H), 2.38-2.3 (m, 4H), 2.17-2.11 (m, 1H), 1.95-1.89 (m, 1H), 1.84-1.78 (m, 2H), 1.28-1.24 (t, 3H, J=7.2Hz).

(S)-6-(2-Azidomethyl-morpholin-4-yl)-butyric acid ethyl ester **6a** (442 mg, 1.73 mmol) and 45 mg of palladium on activated carbon were added to a reaction flask, and the reaction mixture was purged with hydrogen for three time and added with 20 mL of methanol with stirring. The reaction mixture was reacted overnight under hydrogen atmosphere and monitored by thin layer chromatography until the disappearance of the starting materials. The mixture was filtered and concentrated to obtain the title compound (R)-6-(2-aminomethyl-morpholin-4-yl)-butyric acid ethyl ester **6b** as a light yellow liquid for direct use in the next step.
MS m/z (ESI): 231.2 [M+1].

(R)-6-(2-Aminomethyl-morpholin-4-yl)-butyric acid ethyl ester **6b** (0.199 g, 0.865 mmol) was dissolved in 20 mL of dichloromethane with stirring, and added with 4-amino-5-chloro-2-methoxy-benzoic acid (0.209 g, 1.038 mmol) and 1-ethyl-3-(3-dimethyl-propyl)carbodiimide hydrochloride (0.332 g, 1.73 mmol). The reaction mixture was stirred for 3 hours at room temperature and monitored by thin layer chromatography until the disappearance of the starting materials. The mixture was added with 100 mL of saturated sodium bicarbonate solution, and extracted with ethyl acetate (200 mL×3). The combined organic phase was washed with saturated brine (100 mL), dried over anhydrous magnesium sulfate, filtered and concentrated under reduced pressure. The residue was purified by silica gel column chromatography to obtain the title compound (R)-5-{2-[(4-amino-5-chloro-2-methoxy-benzoylamino)-methyl]-morpholin-4-yl}-butyric acid ethyl ester **6c** (0.25 g, yield 69.8%) as a colorless transparent viscous solid.
MS m/z (ESI): 414.2 [M+1].

(R)-5-{2-[(4-Amino-5-chloro-2-methoxy-benzoylamino)-methyl]-morpholin-4-yl}-butyric acid ethyl ester **6c** (0.25 g, 0.605 mmol) was dissolved in 20 mL of toluene with stirring, and added with 1-aza-bicyclo[2.2.2]octan-3-ol (0.384 g, 3.025 mmol). Tetraethyl titanate (0.207 g, 0.908 mmol) was added dropwise at room temperature. The reaction mixture was heated to reflux overnight and monitored by thin layer chromatography until the disappearance of the starting materials. The reaction mixture was concentrated to remove toluene, added with dichloromethane (50 mL) and saturated sodium bicarbonate solution (50 mL), and extracted with dichloromethane (100 mL×3). The combined organic phase was washed with saturated brine (100 mL), dried over anhydrous magnesium sulfate, filtered and concentrated under reduced pressure. The residue was purified by silica gel column chromatography to obtain the title compound (R)-quinuclidin-3-yl 5-((R)-2-((4-amino-5-chloro-2-ethoxybenzamido)methyl)morpholino)butanoate **6** (40 mg, yield 13.4%) as a light yellow solid.
MS m/z (ESI): 495.2 [M+1].
¹H NMR (CDCl₃, 400 MHz) δ 8.09 (s, 1H), 7.89 (s, 1H), 6.28 (s, 1H), 4.79-4.77 (m, 1H), 4.41 (s, 2H), 3.89-3.88 (s, 3H), 3.89-3.88 (m, 1H), 3.68-3.66 (m, 3H), 3.40-3.2 (m, 2H), 2.8-2.6 (m, 7H), 2.38-2.33 (m, 4H), 2.118-2.11 (m, 1H), 1.98-1.97 (m, 2H). 1.95-1.91 (m, 1H), 1.84-1.78 (m, 3H), 1.70-1.35 (m, 3H).

### Example 7

### (R)-Quinuclidin-3-yl 5-((R)-2-((4-amino-5-chloro-2-ethoxybenzamido)methyl)morpholino)pentanoate

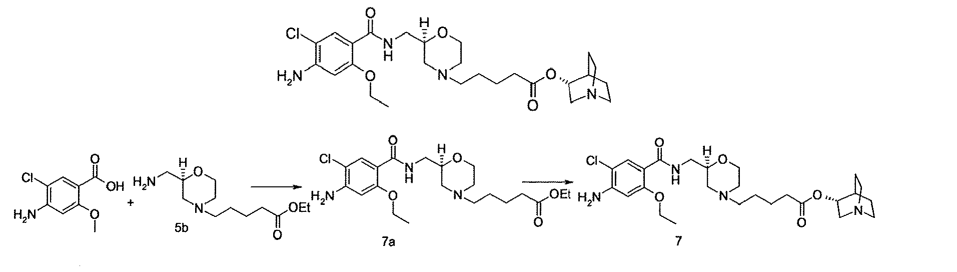

(R)-6-(2-Aminomethyl-morpholin-4-yl)-pentanoic acid ethyl ester **5b** (0.242 g, 1.0 mmol) was dissolved in 20 mL of dichloromethane with stirring, and added with 4-amino-5-chloro-2-ethoxy-benzoic acid (0.259 g, 1.2 mmol) and 1-ethyl-3-(3-dimethyl-propyl)carbodiimide hydrochloride (0.383 g, 2.0 mmol). The reaction mixture was stirred for 3 hours at room temperature and monitored by thin layer chromatography until the disappearance of the starting materials. The mixture was added with 100 mL of saturated sodium bicarbonate solution, and extracted with ethyl acetate (200 mL×3). The combined organic phase was washed with saturated brine (100 mL), dried over anhydrous magnesium sulfate, filtered and concentrated under reduced pressure. The residue was purified by silica gel column chromatography to obtain the title compound (R)-5-{2-[(4-amino-5-chloro-2-ethoxy-benzoylamino)-methyl]-morpholin-4-yl}-pentanoic acid ethyl ester **7a** (0.276 g, yield 62.7%) as a white viscous liquid.
MS m/z (ESI): 442.2 [M+1].

(R)-5-{2-[(4-Amino-5-chloro-2-ethoxy-benzoylamino)-methyl]-morpholin-4-yl}-pentanoic acid ethyl ester **7a** (0.22 g, 0.5 mmol) was dissolved in 20 mL of toluene with stirring, and added with 1-aza-bicyclo[2.2.2]octan-3-ol (0.318 g, 2.5 mmol). Tetraethyl titanate (0.171 g, 0.75 mmol) was added dropwise at room temperature. The reaction mixture was heated to reflux overnight and monitored by thin layer chromatography until the disappearance of the starting materials. The reaction mixture was concentrated to remove toluene, added with dichloromethane (50 mL) and saturated sodium bicarbonate solution (50 mL), and extracted with dichloromethane (100 mL×3). The combined organic phase was washed with saturated brine (100 mL), dried over anhydrous magnesium sulfate, filtered and concentrated under reduced pressure. The residue was purified by silica gel column chromatography to obtain the title compound (R)-quinuclidin-3-yl 5-((R)-2-((4-amino-5-chloro-2-ethoxybenzamido)methyl)morpholino)pentanoate 7 (30 mg, yield 11.5%) as a light yellow solid.
MS m/z (ESI): 523.3 [M+1].
¹H NMR (CDCl₃, 400 MHz) δ 8.22 (s, 1H), 8.09 (s, 1H), 6.29 (s, 1H), 4.87-4.85 (m, 1H), 4.41 (s, 2H), 4.09-4.05 (q, 2H, J=7.2Hz), 3.9-3.86 (m, 1H), 3.72-3.65 (m, 3H), 3.38-3.29 (m, 4H), 3.00-2.26 (m, 7H), 2.37-2.33 (m, 4H), 2.15-2.10 (m, 2H), 1.97-1.81 (m, 2H), 1.80-1.71 (m, 1H), 1.71-1.61 (m, 3H), 1.58-1.47 (t, 3H, J=7.2Hz), 1.58-1.47 (m, 3H).

### Example 8

### (R)-Quinuclidin-3-yl 5-((R)-2-((4-amino-5-chloro-2-ethoxybenzamido)methyl)morpholino)butanoate

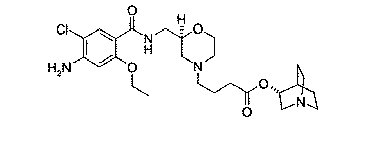

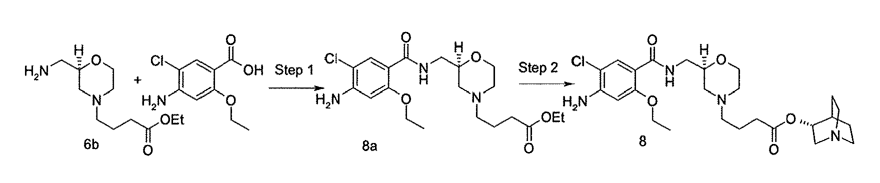

(R)-6-(2-Aminomethyl-morpholin-4-yl)-butyric acid ethyl ester **6b** (0.199 g, 0.865 mmol) was dissolved in 20 mL of dichloromethane with stirring, and added with 4-amino-5-chloro-2-ethoxy-benzoic acid (0.209 g, 1.038 mmol) and 1-ethyl-3-(3-dimethyl-propyl)carbodiimide hydrochloride (0.332 g, 1.73 mmol). The reaction mixture was stirred for 3 hours at room temperature and monitored by thin layer chromatography until the disappearance of the starting materials. The mixture was added with 100 mL of saturated sodium bicarbonate solution, and extracted with ethyl acetate (200 mL×3). The combined organic phase was washed with saturated brine (100 mL), dried over anhydrous magnesium sulfate, filtered and concentrated under reduced pressure. The residue was purified by silica gel column chromatography to obtain the title compound (R)-5-{2-[(4-amino-5-chloro-2-ethoxy-benzoylamino)-methyl]-morpholin-4-yl}-butyric acid ethyl ester **8a** (0.25 g, yield 69.8%) as a colorless transparent viscous solid.
MS m/z (ESI): 414.2 [M+1].

(R)-5-{2-[(4-Amino-5-chloro-2-ethoxy-benzoylamino)-methyl]-morpholin-4-yl}-butyric acid ethyl ester **8a** (0.235 g, 0.55 mmol) was dissolved in 20 mL of toluene with stirring, and added with 1-aza-bicyclo[2.2.2]octan-3-ol (0.35 g, 2.75 mmol). Tetraethyl titanate (0.188 g, 0.825 mmol) was added dropwise at room temperature. The reaction mixture was heated to reflux overnight and monitored by thin layer chromatography until the disappearance of the starting materials. The reaction mixture was concentrated to remove toluene, added with dichloromethane (50 mL) and saturated sodium bicarbonate solution (50 mL), and extracted with dichloromethane (100 mL×3). The combined organic phase was washed with saturated brine (100 mL), dried over anhydrous magnesium sulfate, filtered and concentrated under reduced pressure. The residue was purified by silica gel column chromatography to obtain the title compound (R)-quinuclidin-3-yl 5-((R)-2-((4-amino-5-chloro-2-ethoxybenzamido)methyl)morpholino)butanoate **8** (51 mg, yield 18.3%) as a light yellow solid.
MS m/z (ESI): 509.4 [M+1].
¹H NMR (CDCl₃, 400 MHz) δ 8.22 (s, 1H), 8.08 (s, 1H), 6.30 (s, 1H), 5.01-4.99 (m, 1H), 4.43 (m, 2H), 4.11-4.06 (q, 2H, J=6.8Hz), 3.89-3.86 (m, 1H), 3.72-3.64 (m, 3H), 3.55-3.3 (m, 2H), 3.21-3.02 (m, 5H), 2.78-2.75 (d, 1H, J=3.2Hz), 2.69-2.66 (d, 1H, J=3.6Hz), 2.42-2.34 (m, 4H), 2.16-2.12 (m, 1H), 1.96-1.91 (m, 2H), 1.85-1.76 (m, 5H), 1.51-1.48 (t, 3H, J=6.8Hz).

### Example 9

### (R)-Quinuclidin-3-yl 5-((R)-2-((4-amino-5-chloro-2-ethoxybenzamido)methyl)morpholino)hexanoate

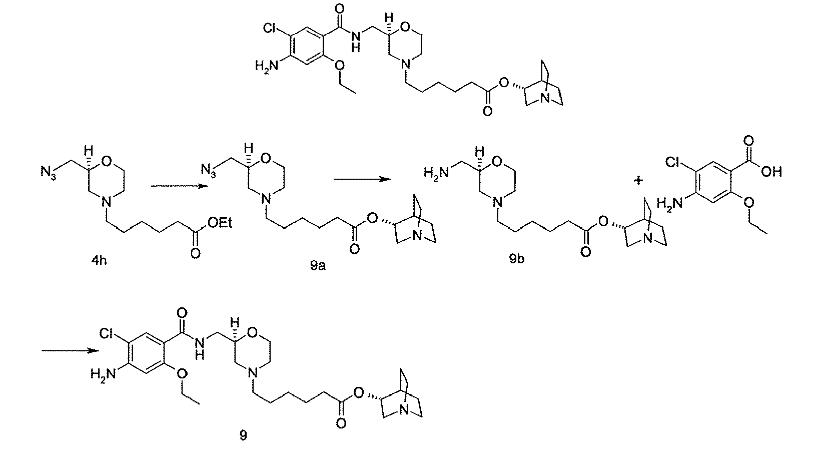

(S)-6-(2-Azidomethyl-morpholin-4-yl)-hexanoic acid ethyl ester **4h** (2.84 g, 10.0 mmol) was dissolved in 140 mL of toluene with stirring, and added with tetraethyl titanate (2.28 g, 10 mmol) and 1-aza-bicyclo[2.2.2]octan-3-ol (5.08 g, 40 mmol). The reaction mixture was heated to 90°C overnight and monitored by thin layer chromatography until the product formed. There was still part of starting materials left. The mixture was added with 100 mL of saturated sodium bicarbonate solution, and extracted with ethyl acetate (400 mL×3). The combined organic phase was washed successively with water (200 mL) and saturated brine (200 mL), dried over anhydrous magnesium sulfate, filtered and concentrated under reduced pressure. The residue was purified by silica gel column chromatography to obtain the title compound (R)-quinuclidin-3-yl 6-((S)-2-(azidomethyl)morpholin)-hexanoate **9a** (3.252 g, yield 89%) as a light yellow oil.
¹H NMR (CDCl₃, 400 MHz) δ 4.82-4.79 (m, 1H), 3.9-3.89 (m, 1H), 3.70-3.67 (m, 3H), 3.3-3.2 (m, 2H), 2.90-2.87 (m, 2H), 2.79-2.78 (m, 2H), 2.72-2.66 (m, 3H), 2.35-2.31 (m, 4H), 2.12-2.07 (m, 1H), 2.02-2.00 (m, 1H), 1.93-1.88 (m, 2H), 1.69-1.63 (m, 3H), 1.53-1.49 (m, 3H), 1.39-1.35 (m, 3H).

(R)-Quinuclidin-3-yl 6-((S)-2-(azidomethyl)morpholin)-hexanoate **9a** (1.802 g, 4.93 mmol) was dissolved in 100 mL of isopropyl alcohol with stirring, and added with palladium on activated carbon (0.18 g, 0.493 mmol). The reaction mixture was purged with hydrogen for three times, reacted overnight at room temperature under hydrogen atmosphere and monitored by thin layer chromatography until the substantial disappearance of the starting materials. The mixture was filtered to remove palladium on activated carbon and the solvent was evaporated to obtain the crude product of the title compound (R)-quinuclidin-3-yl 6-((R)-2-aminomethyl-morpholin) hexanoate **9b** (2.178 g) as a light yellow liquid for direct use in the next step.
MS m/z (ESI): 340.4 [M+1].

(R)-Quinuclidin-3-yl 6-((R)-2-aminomethyl-morpholin) hexanoate **9b** (1.67 g, 4.93 mmol) was dissolved in 100 mL of dichloromethane with stirring, and added with 4-amino-5-chloro-2-ethoxy-benzoic acid (1.17 g, 5.42 mmol) and 1-ethyl-3-(3-dimethyl-propyl)carbodiimide hydrochloride(1.89 g, 9.86 mmol). The reaction mixture was stirred for 3 hours at room temperature and monitored by thin layer chromatography until the disappearance of the starting materials. The mixture was quenched with 100 mL of saturated sodium bicarbonate solution, and extracted with dichloromethane (400 mL×3). The combined organic phase was washed successively with water (300 mL) and saturated brine (300 mL), dried over anhydrous magnesium sulfate, filtered and concentrated under reduced pressure. The residue was recrystallized from n-hexane/ethyl acetate to obtain the title compound (R)-quinuclidin-3-yl
5-((R)-2-((4-amino-5-chloro-2-ethoxybenzamido)methyl)morpholino)hexanoate **9** (2.47 g, yield 93.4%) as a white solid.
MS m/z (ESI): 537.3 [M+1].
¹H NMR (CDCl₃, 400 MHz) δ 8.2 (s, 1H), 8.1 (s, 1H), 6.27 (s, 1H), 4.8 (m, 1H), 4.40 (s, 2H), 4.1-4.05 (q, 2H, J=6.8Hz), 3.88 (d, 1H, J=6.4Hz), 3.71-3.66 (m, 3H), 3.37-3.31 (m, 1H), 3.28-3.22 (m, 1H), 2.92-2.68 (m, 7H), 2.34-2.3 (m, 4H), 2.15-2.08 (m, 1H), 2.02-2.01 (m, 1H), 1.93-1.86 (m, 2H), 1.74-1.69 (m, 3H), 1.49-1.47 (m, 3H), 1.49-1.47 (t, 3H, J=6.8Hz), 1.4-1.3 (m, 3H).

### Example 10

### (R)-6-(2-((4-Amino-5-chloro-2-ethoxybenzamido)methyl)morpholino)hexanoic acid

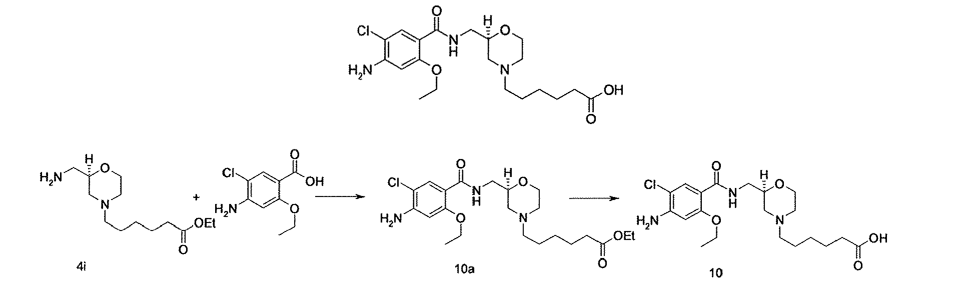

(R)-6-(2-Aminomethyl-morpholin-4-yl)-hexanoic acid ethyl ester **4i** (3.27 g, 12.68 mmol) was dissolved in 100 mL of dichloromethane with stirring, and added with 4-amino-5-chloro-2-ethoxy-benzoic acid (3.01 g, 13.95 mmol) and 1-ethyl-3-(3-dimethyl-propyl)carbodiimide hydrochloride(6.08 g, 31.7 mmol). The reaction mixture was stirred for 5 hours at room temperature and monitored by thin layer chromatography until the disappearance of the starting materials. The mixture was quenched with 100 mL of saturated sodium bicarbonate solution, and extracted with dichloromethane (500 mL×3). The combined organic phase was washed successively with water (300 mL) and saturated brine (300 mL), dried over anhydrous magnesium sulfate, filtered and concentrated under reduced pressure. The residue was purified by silica gel column chromatography to obtain the title compound (R)-6-(2-((4-amino-5-chloro-2-ethoxy-benzoylamino)-methyl)-morpholino)-hexanoic acid ethyl ester **10a** (3.36 g, yield 58%) as a white solid.
MS m/z (ESI): 456.3 [M+1].
¹H NMR (CDCl₃, 400 MHz) δ 8.2 (m, 1H), 8.1 (s, 1H), 6.28 (s, 1H), 4.40 (s, 2H), 4.18-4.06 (m, 4H), 3.9-3.88 (m, 1H), 3.71-3.66 (m, 4H), 3.37-3.34 (m, 1H), 2.81-2.78 (d, 1H, J=11.2Hz), 2.71-2.68 (d, 1H, J=11.6Hz), 2.34-2.27 (m, 4H), 2.15-2.09 (m, 1H), 1.94-1.89 (m, 1H), 1.67-1.6 (m, 2H), 1.51-1.46 (m, 5H), 1.37-1.31 (m, 2H), 1.27-1.23 (m, 3H).
(R)-6-(2-((4-Amino-5-chloro-2-ethoxy-benzoylamino)-methyl)-morpholino)-hexanoic acid ethyl ester **10a** (0.11 g, 0.24 mmol) was dissolved in 10 mL of methanol with stirring, and added with 3 mL of sodium hydroxide solution (15%). The reaction mixture was reacted for 30 minutes at room temperature and monitored by thin layer chromatography until the disappearance of the starting materials. The mixture was adjusted to pH 3-3.5 with glacial acetic acid, and extracted with ethyl acetate (100 mL×3). The combined organic phase was washed with saturated brine (100 mL), filtered and concentrated under reduced pressure. The residue was purified by silica gel column chromatography to obtain the title compound (R)-6-(2-((4-amino-5-chloro-2-ethoxy-benzoylamino)-methyl]-morpholino)-hexanoic acid **10** (40 mg, yield 36%) as a white solid.
MS m/z (ESI): 428.3 [M+1].
¹H NMR (CD₃OD, 400 MHz)δ 7.83 (s, 1H), 6.49 (s, 1H), 4.15 (q, 2H), 3.92 (m, 1H), 3.75-3.55 (m, 4H), 3.25-3.2 (m, 1H), 2.90-2.85 (m, 2H), 2.51 (m, 2H), 2.35-2.25 (m, 1H), 2.21 (m, 2H), 2.10 (m, 2H), 1.68-1.54 (m, 2H), 1.50 (t, 3H), 1.35 (m, 2H).

### Example 11

### (S)-6-(2-((4-Amino-5-chloro-2-ethoxy-benzoylamino)methyl)morpholino)-hexanoic acid

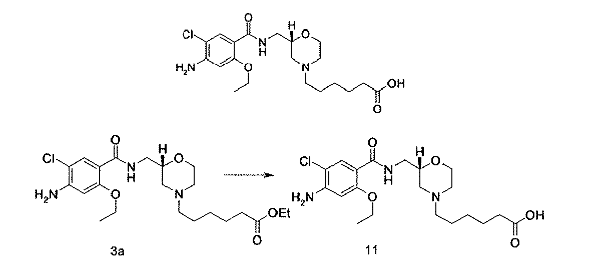

(S)-6-(2-((4-Aamino-5-chloro-2-ethoxy-benzoylamino)methyl)-morpholino)-hexanoic acid ethyl ester **3a** (0.11 g, 0.24 mmol) was dissolved in 10 mL of methanol with stirring, and added with 3 mL of sodium hydroxide solution (15%). The reaction mixture was reacted for 30 minutes at room temperature and monitored by thin layer chromatography until the disappearance of the starting materials. The mixture was adjusted to pH 3-3.5 with glacial acetic acid, and extracted with ethyl acetate (100 mL×3). The combined organic phase was washed with saturated brine (100 mL), filtered and concentrated under reduced pressure. The residue was purified by silica gel column chromatography to obtain the title compound (S) -6-(2-((4-amino-5-chloro-2-ethoxy-benzoylamino)-methyl)-morpholino)-hexanoic acid **11** (40 mg, yield 36%) as a white solid.
MS m/z (ESI): 428.2 [M+1].
¹H NMR (CD₃OD, 400 MHz) δ 7.84 (s, 1H), 6.49 (s, 1H), 4.16-4.14 (q, 2H), 3.93 (m, 1H), 3.75-3.58 (m, 4H), 3.38-3.34 (m, 1H), 2.99-2.88 (m, 2H), 2.52-2.48 (m, 2H), 2.35-2.25 (m, 1H), 2.25-2.22 (m, 2H), 2.09-2.05 (m, 2H), 1.70-1.55 (m, 2H), 1.52-1.48 (t, 3H), 1.39-1.35 (m, 2H).
**Pharmacological Assay:**
5-HT₄ Receptor Agonist Binding Assay
**Purpose**: To determine binding ratio of invention compounds to 5-HT₄ receptor
**Material**: Rat striatal tissue
**Samples**: Positive control mosapride, ATI-7505 and examples 1, 3, 7 and 9
**Reagents**: [³H]-GR113808 (Amersham, TRK944-10UCI, 370kBq, 5µCi, 60-85Ci/mmol, 20µCi/ml)), GR113808 (Sigma, G5918)
**Assay protocol**
1. Tissue sample preparation
a) Rats were killed. Put striatal tissue in 15 volumes of Tris-HCL buffer (50mM, pH 7.4, 4°C) and homogenized, and centrifuged for 10 minutes at 48,000 rpm at 4°C.
b) The resulting precipitate was re-suspended in the Tris-HCL buffer.
c) Determined the protein concentration. Adjusted the protein concentration to 30 mg/ml. The homogenate was aliquoted and stored at -80°C.
d) The Striatal tissue sample should be diluted to 1 mg/ml before use.

2. Dilution of [³H]-GR113808 isotope stock solution
The concentration of [³H]-GR113808 stock solution is calculated to be 0.23∼0.33µM. The concentration of assay working solution of [³H]-GR113808 is 0.16nM. The process for diluting the stored solution by 10x is to add 10 µl stock solution to 1.8 ml Tris-HCL buffer (50 mM, pH 7.4).
3. Preparation of 10x testing compounds (gradient concentration) solution The testing compounds were firstly dissolved in the Tris-HCL buffer to give a concentration of 25 mM, then the compounds were diluted to a serial gradient concentration with 5 times dilution ratio to make a 10x testing solutions (5⁻⁷, 5⁻⁶, 5⁻⁵, 5⁻⁴, 5⁻³, 5⁻², 5⁻¹, 5⁰, 5, 5² mM).
4. Competitive binding assay Place the assay tubes on ice and add each solution (All reactions were performed in triplicate; unit: µl; total volume of the reaction tube is 200 µl).
5. Results
The assay results were shown in Table 1.
Based on the results of testing compounds, the invention compounds can bind to the 5-HT₄ receptor.

**Table 1. The result of binding ratio of 5-HT₄ agonist compounds**

| | Structure | IC₅₀(nM) |
|---|---|---|
| Mosapride positive control | 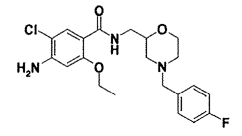 | 298 |
| ATI 7505 positive control | 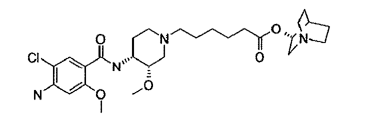 | 22.73 |
| Example 1 | 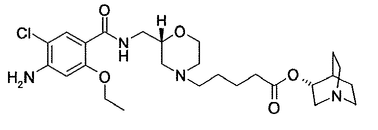 | 73.85 |
| Example 3 | 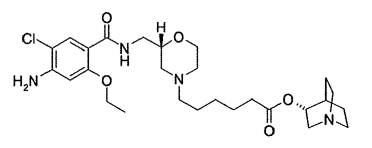 | 24.43 |
| Example 7 | 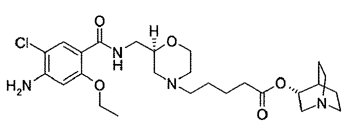 | 240 |
| Example 9 | 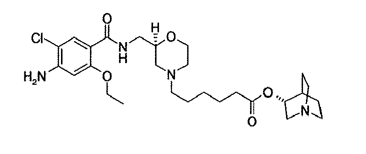 | 1774 |

## Claims

1. Compounds having the formula (I) or pharmaceutically acceptable salts thereof: 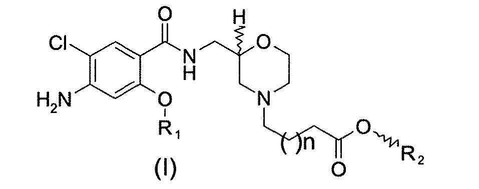 wherein:
R₁ is alkyl;
R₂ is selected from the group consisting of hydrogen, alkyl, cycloalkyl and heterocyclo alkyl; and
n is an integer from 1 to 5.

2. The compounds or pharmaceutically acceptable salts thereof of claim 1, wherein R₁ is methyl, ethyl or isopropyl.

3. The compounds or pharmaceutically acceptable salts thereof of claim 1 or claim 2, wherein R₂ is hydrogen, alkyl or heterocyclo alkyl.

4. The compounds or pharmaceutically acceptable salts thereof of claim 1 or claim 2, wherein n is an integer from 1 to 3.

5. The compounds or pharmaceutically acceptable salts thereof of claim 1 or claim 2, wherein R₂ is

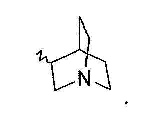

6. The compounds or pharmaceutically acceptable salts thereof of claim 1 or claim 2, including the compounds having the formula (IA) or pharmaceutically acceptable salts thereof:

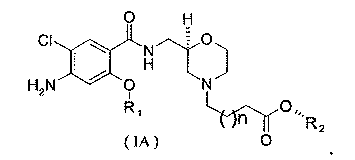

7. The compounds or pharmaceutically acceptable salts thereof of claim 1 or claim 2, including the compounds having the formula (IB) or pharmaceutically acceptable salts thereof:

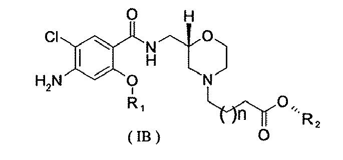

8. The compounds or pharmaceutically acceptable salts thereof of claim 1 or claim 2, wherein said salts are those formed with the acids selected from the group consisting of hydrochloric acid, methanesulfonic acid, sulfuric acid, tartaric acid, citric acid, acetic acid and trifluoroacetic acid.

9. The compounds or pharmaceutically acceptable salts thereof of claim 1, including:

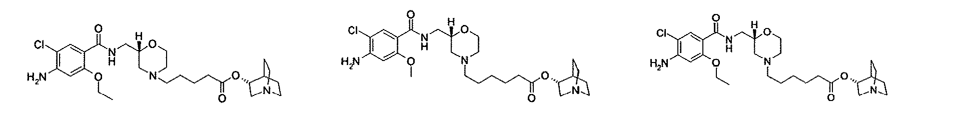

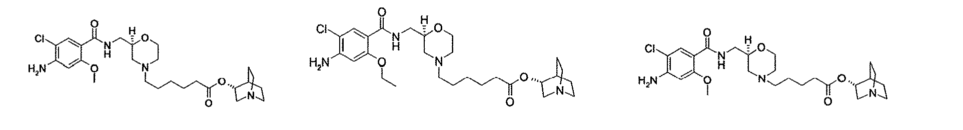

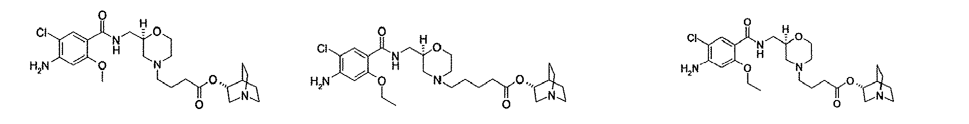

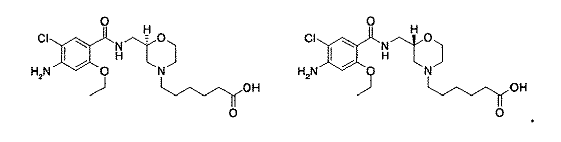

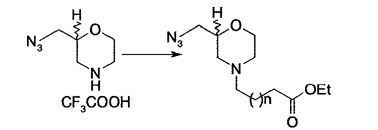

10. Coupling azidomethyl-morpholine trifluoroacetate with Br-CH₂-(CH₂)n-CH₂-C(O)OEt under alkaline condition to obtain N-substituted azidomethyl-morpholine; 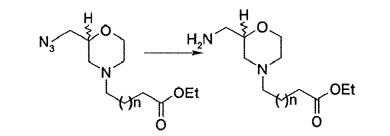 reducing the obtained N-substituted azidomethyl-morpholine by hydrogenation to obtain N-substituted aminomethyl-morpholine; 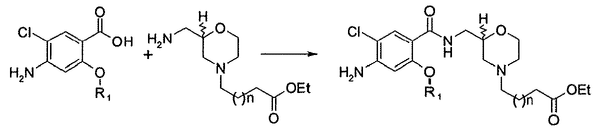 reacting the N-substituted aminomethyl-morpholine with substituted benzoic acid in the presence of the condensation reagent 1-ethyl-3-(3-dimethyl-propyl)carbodiimide hydrochloride at room temperature to obtain benzamide derivatives; 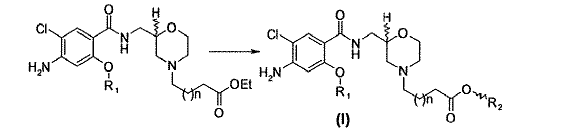 reacting the benzamide derivatives in toluene with R₂-OH by transesterification, or hydrolyzing under alkaline condition to obtain compounds having the formula (I).

11. A process for preparing the compound having the formula (I) of claim 1, comprising: 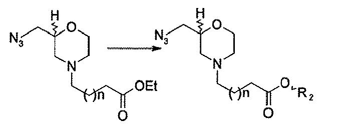 reacting N-substituted azidomethyl-morpholine in toluene with R₂-OH by transesterification to obtain the transesterification product; 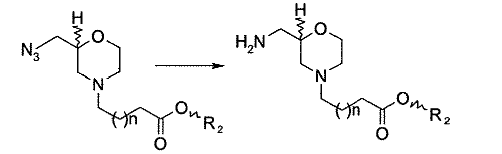 reducing the transesterification product by hydrogenation to obtain N-substituted aminomethyl-morpholine; 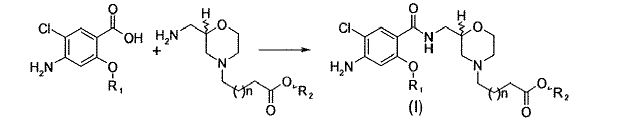 reacting the N-substituted aminomethyl-morpholine with substituted benzoic acid in the presence of the condensation reagent 1-ethyl-3-(3-dimethyl-propyl)carbodiimide hydrochloride at room temperature, or further hydrolyzing under alkaline condition to obtain the compounds having the formula (I).

12. A pharmaceutical composition comprising the compounds or pharmaceutically acceptable salts thereof of any one of claims 1-9 in an effective therapeutic amount, as well as pharmaceutically acceptable carrier.

13. A use of the compounds or pharmaceutically acceptable salts thereof of claim 1 in the preparation of a medicament for the treatment of the diseases selected from the group consisting of gastroesophageal reflux disease, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

14. A use of the compounds or pharmaceutically acceptable salts thereof of claim 1 in the preparation of a medicament for the treatment of the diseases of mammals mediated by 5-HT₄ receptor.

15. The use of the composition of claim 12 in the preparation of a medicament for the treatment of the diseases selected from the group consisting of gastroesophageal reflux disease, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

16. The use of the composition of claim 12 in the preparation of a medicament for the treatment of the diseases of mammals mediated by 5-HT₄ receptor.

## Amended claims

### Amended claims under Art. 19.1 PCT

**1.** Compounds having the formula (I) or pharmaceutically acceptable salts thereof 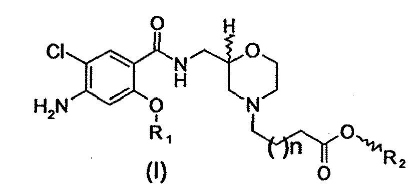 wherein:
R₁ is alkyl;
R₂ is 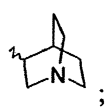 and
n is an integer from 1 to 5.

**2.** The compounds or pharmaceutically acceptable salts thereof of claim 1, wherein R₁ is methyl, ethyl or isopropyl.

**3.** The compounds or pharmaceutically acceptable salts thereof of claim 1 or claim 2, wherein n is an integer from 1 to 3.

**4.** The compounds or pharmaceutically acceptable salts thereof of claim 1 or claim 2, including the compounds having the formula (IA) or pharmaceutically acceptable salts thereof: 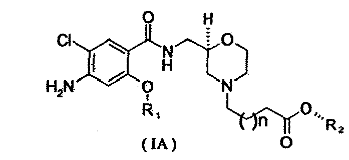 wherein R₁, R₂, n are the same as defined in claim 1.

**5.** The compounds or pharmaceutically acceptable salts thereof of claim 1 or claim 2, including the compounds having the formula (IB) or pharmaceutically acceptable salts thereof: 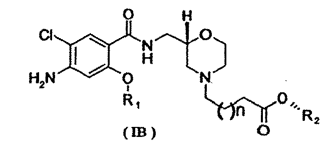 wherein R₁, R₂, n are the same as defined in claim 1.

**6.** The compounds or pharmaceutically acceptable salts thereof of claim 1 or claim 2, wherein said salts are those formed with the acids selected from the group consisting of hydrochloric acid, methanesulfonic acid, sulfuric acid, tartaric acid, citric acid, acetic acid and trifluoroacetic acid.

**7.** The compounds or pharmaceutically acceptable salts thereof of claim 1, including:

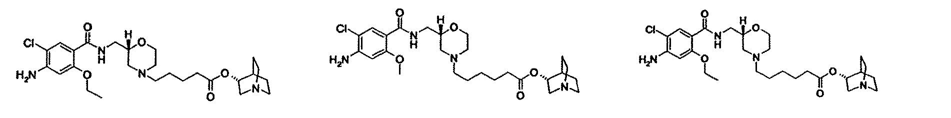

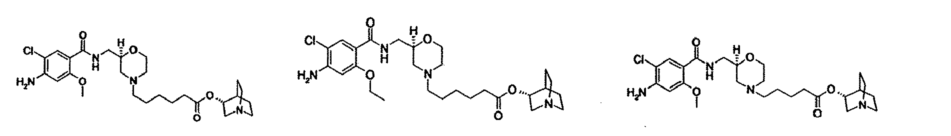

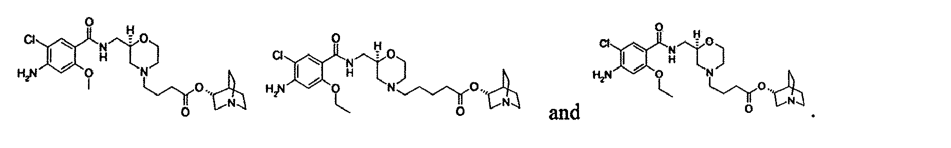

**8.** A process for preparing the compound having the formula (I) of claim 1, comprising: 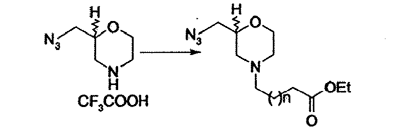 coupling azidomethyl-morpholine trifluoroacetate with Br-CH₂-(CH₂)n-CH₂-C(O)OEt under alkaline conditions to obtain N-substituted azidomethyl-morpholine; 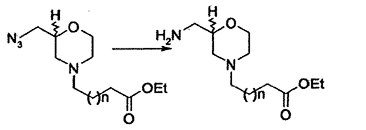 reducing the obtained N-substituted azidomethyl-morpholine by hydrogenation to obtain N-substituted aminomethyl-morpholine; 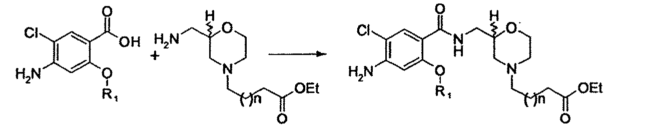 reacting the N-substituted aminomethyl-morpholine with substituted benzoic acid in the presence of the condensation reagent 1-ethyl-3-(3-dimethyl-propyl)carbodiimide hydrochloride at room temperature to obtain benzamide derivatives; 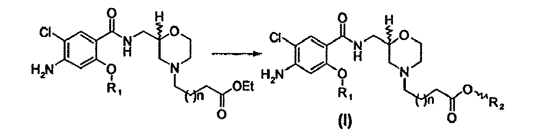 reacting the benzamide derivatives in toluene with R₂-OH by transesterification, or hydrolyzing under alkaline conditions to obtain compounds having the formula (I).

**9.** A process for preparing the compound having the formula (I) of claim 1, comprising: 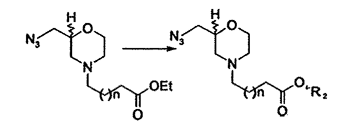 reacting N-substituted azidomethyl-morpholine in toluene with R₂-OH by transesterification to obtain the transesterification product; 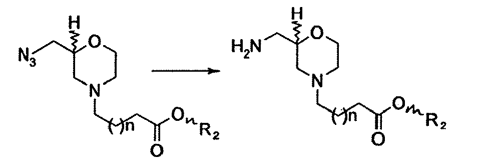 reducing the transesterification product by hydrogenation to obtain N-substituted aminomethyl-morpholine; 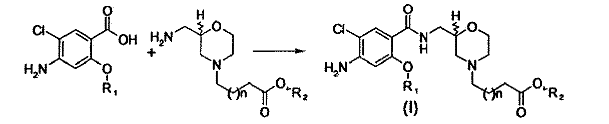 reacting the N-substituted aminomethyl-morpholine with substituted benzoic acid in the presence of the condensation reagent 1-ethyl-3-(3-dimethyl-propyl)carbodiimide hydrochloride at room temperature, or further hydrolyzing under alkaline conditions to obtain compounds having the formula (I).

**10.** A pharmaceutical composition comprising compounds or pharmaceutically acceptable salts thereof of any one of claims 1-7 in an effective therapeutic amount, as well as pharmaceutically acceptable carrier.

**11.** A use of the compounds or pharmaceutically acceptable salts thereof of claim 1 in the preparation of a medicament for the treatment of the diseases selected from the group consisting of gastroesophageal reflux disease, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

**12.** A use of the compounds or pharmaceutically acceptable salts thereof of claim 1 in the preparation of a medicament for the treatment of the diseases of mammals mediated by 5-HT₄ receptor.

**13.** The use of the composition of claim 10 in the preparation of a medicament for the treatment of the diseases selected from the group consisting of gastroesophageal reflux disease, gastrointestinal diseases, gastric motility obstacles, non-ulcer dyspepsia, functional dyspepsia, irritable bowel syndrome, constipation, indigestion, esophagitis, gastric esophageal disease, nausea, postoperative intestinal infarction, central nervous system disease, Alzheimer's disease, cognitive impairment, vomiting, migraine, neurological diseases, pain, cardiovascular disease, heart failure, arrhythmias, intestinal pseudo-obstruction, gastroparesis, diabetes and apnea syndrome.

**14.** The use of the composition of claim 10 in the preparation of a medicament for the treatment of the diseases of mammals mediated by 5-HT₄ receptor.
